# EUROPEAN PATENT APPLICATION

(11) **EP 1 208 850 A1**
(43) Date of publication of application: **29.05.2002**
(21) Application number: 00949995.5
(22) Date of filing: 04.08.2000
(51) Int. Cl.: A61K 45/00, A61K 31/67, A61K 9/10, A61K 47/04, A61K 47/36, A61P 3/14, A61P 19/08

(54) **PASTES WITH THE SUSTAINED RELEASE OF OSTEOGENESIS PROMOTER**

(30) Priority: 05.08.1999 JP 22306299
(71) Applicant: Takeda Chemical Industries, Ltd., Osaka-shi, Osaka 541-0045 (JP)
(72) Inventor: HOSHINO, Tetsuo, Toyono-gun, Osaka 563-0105 (JP); MAKINO, Haruhiko, Kawabe-gun, Hyogo 666-0251 (JP); KURASAWA, Takashi, Kyoto-shi, Kyoto 606-0837 (JP)
(74) Representative: Lewin, John Harvey
(86) International application number: JP0005239
(87) International publication number: WO0110463

(57) **Abstract**

The invention provides an osteogenesis promoter sustained-release paste, which comprises an osteogenesis promoter, a calcium component and a viscosity-increasing agent, which has a high tissue affinity and adherability to a bone and can release gradually an osteogenesis promoter constantly for a long period, and as a result, which can be used conveniently in order to promote coaptation between bones and repairment of a defect site in treating a fracture.

## Description

### Technical Field

The present invention relates to an osteogenesis promoter sustained-release paste. More specifically, the present invention relates to an osteogenesis promoter sustained-release paste, which comprises an osteogenesis promoter, a calcium component and a viscosity-increasing agent.

### Background Art

Presently, a formulation technology is aggressively researched to release an active component gradually at a desired site after *in vivo* administration. Particularly, in a fracture treatment, since a fracture site is covered by a plaster and the like, it is often difficult to administer a drug several times. Therefore, development of a sustained-release preparation capable of providing a drug to a fracture site at a high concentration for a long period only by a single administration is greatly expected.

In this situation, as shown in, for example, JP-A 9-263545, a sustained-release preparation has been proposed which comprises a benzothiepine derivative, a non-peptide low molecular weight compound having an excellent osteogenetic promotion effect, as an active component. This preparation consists of a suspension of a biodegradable polymer containing an active component, and it can be easily applied via an injection and can provide the active component to a desired site such as a fracture site for a certain period at a high concentration. Therefore, the preparation is very useful, and its clinical application is expected. However, since this preparation is a liquid and has high fluidity, when it is applied to an open fracture, retention at the administered site is not necessarily sufficient.

On the other hand, an artificial bone and bone filling materials having a high affinity to bone tissues are conventionally known, and a technology has been proposed to incorporate a variety of active components into these artificial bone and bone filling materials.

For example, JP-A 6-228011 describes a calcium phosphate type drug sustained-release material and a process for manufacturing it. This drug sustained-release material binds to a biological hard tissue and remains therein, and release rate of the drug can be easily controlled. However, since the matrix used therein consists of only so-called self-setting cements, tetracalcium phosphate and dicalcium phosphate, spreadability, an application property and adherability of the matrix to the hard tissue are poor in a state of slurry or clay before the completion of setting, and, consequently, it is probable that the drug sustained-release material is disintegrated and washed away by contacting with a body fluid such as blood.

In addition, JP-A 7-31673 discloses a bone filling material consisting of a self-setting cement and a natural bioabsorbable polymer material, such as dextran, and a physiologically active material which may be incorporated into this bone filling material includes, for example, an osteogenetic factor, a bone growth factor and the like. JP-A 7-289627 discloses a setting composition for a medical treatment which has excellent biocompatibility, and has a form imparting property, does not disintegrate even when the kneaded material thereof right after kneading contacts with a body fluid, wherein a phosphate component, a calcium component and alginic acid compound are incorporated therein. JP-A 10-216219 discloses a setting composition for a medical treatment which has excellent biocompatibility, and has a form imparting property, does not disintegrate even when the kneaded material thereof right after kneading contacts with a body fluid, and exhibits excellent characteristics, such as tacky adhesiveness to the bone, wherein collagen are incorporated therein. Since the main object of these compositions disclosed in the above prior arts are to apply them to a bone defect site so that the setting composition thereof function as a bone supplementation material, the setting composition has a sufficient mechanical strength. However, since the bioactive material incorporated is perfectly contained within the setting composition by setting, although the incorporated bioactive material is to some extent released at the initial stage of setting, it can not be expected that it is constantly released for a long period. In addition, Norian SRS (TRADE NAME, developed by Norian Corporation) as a medical material for treating a fracture, and Biopex (TRADE NAME, applied by Welfide Corporation) as a pasty bone filling material are commercially available, but they contain no bioactive material such as an osterogenesis promoter, and set quickly to impart a sufficient strength after setting. Therefore, even if a bioactive material is contained in them, a constant sustained-release over a long period can not be expected, as discussed above.

As described above, currently, a sustained-release preparation has not been developed which is excellent in spreadability, an application property and adherability, and plasticity, which can remain at a bone tissue by a local administration, and which has a constant sustained releasability over a long period.

In the circumstances, it has been desired to develop a novel preparation having a high tissue affinity and adherability to a bone, having an appropriate hardness so as not to disintegrate when contacted with a body fluid while maintains a certain plasticity, and which can release gradually an osteogenesis promoter constantly over a long period, and, as a result, which can be used conveniently in order to promote coaptation between bones and repair of a defect site in treating a fracture.

### Disclosure of Invention

The present inventors worked diligently to solve the above problem, and, consequently, developed a pasty base material having moderate plasticity and adherability by imparting a base material with a local retention due to a hydraulic setting property of a calcium component, and by incorporating a viscosity-increasing agent therein. Then, the inventors confirmed that the pasty base material can release gradually an osteogenesis promoter over a long period by incorporating it. Thus, the present invention has been completed.

That is, the invention provides:
(1) An osteogenesis promoter sustained-release paste, which comprises an osteogenesis promoter, a calcium component and a viscosity-increasing agent;
(2) The paste according to the above item (1), wherein the calcium component is selected from a group consisting of calcium phosphate, sulfate and carbonate, and any combination thereof;
(3) The paste according to the above item (1), wherein the calcium component is a combination of an anhydrate or a dihydrate of dicalcium phosphate and tetracalcium phosphate;
(4) The paste according to the above item (1), wherein the calcium component is a hemihydrate of calcium sulfate;
(5) The paste according to the above item (4), which further comprises a dihydrate of calcium sulfate;
(6) The paste according to the above item (1), wherein the calcium component is incorporated in about 1-500 parts relative to a part of the osteogenesis promoter by weight;
(7) The paste according to the above item (1), wherein the viscosity-increasing agent is a biocompatible polymer;
(8) The paste according to the above item (1), wherein the viscosity-increasing agent is dextran;
(9) The paste according to the above item (1), wherein the viscosity-increasing agent is incorporated in about 0.05-4 parts relative to a part of the calcium component by weight;
(10) The paste according to the above item (1), wherein the osteogenesis promoter is a non-peptide low-molecular-weight compound;
(11) The paste according to the above item (1); wherein the osteogenesis promoter is benzothiopyran or a benzothiepine derivative;
(12) The paste according to the above item (1), wherein the osteogenesis promoter is (2*R*,4*S*)-(-)-*N*-[4-(diethoxyphosphorylmethyl)phenyl] -1,2,4,5-tetrahydro-4-methyl-7,8-methylenedioxy-5-oxo-3-benzothiepine-2-carboxamide or a salt thereof, or a sustained-release preparation thereof;
(13) The paste according to the above item (1), wherein the osteogenesis promoter is formulated so as to have sustained-releasability with a biodegradative polymer;
(14) A composition for producing the paste according to the above item (1), which comprises a calcium component and a viscosity-increasing agent;
(15) A composition for producing the paste according to the above item (1), which comprises an osteogenesis promoter or a sustained-release preparation thereof, a calcium component and a viscosity-increasing agent;
(16) A process for producing an osteogenesis promoter sustained-release paste, which comprises mixing an osteogenesis promoter, a calcium component, a viscosity-increasing agent and water;
(17) The paste according to any one of the above items (1) to (13), which is a bone disease treatment agent;
(18) A process for treating a bone disease, which comprises administering the paste according to any one of the above items (1) to (13); and
(19) Use of the composition according to the above item (14) or (15) for the manufacture of a bone disease treating agent.

### Brief Description of the Drawings

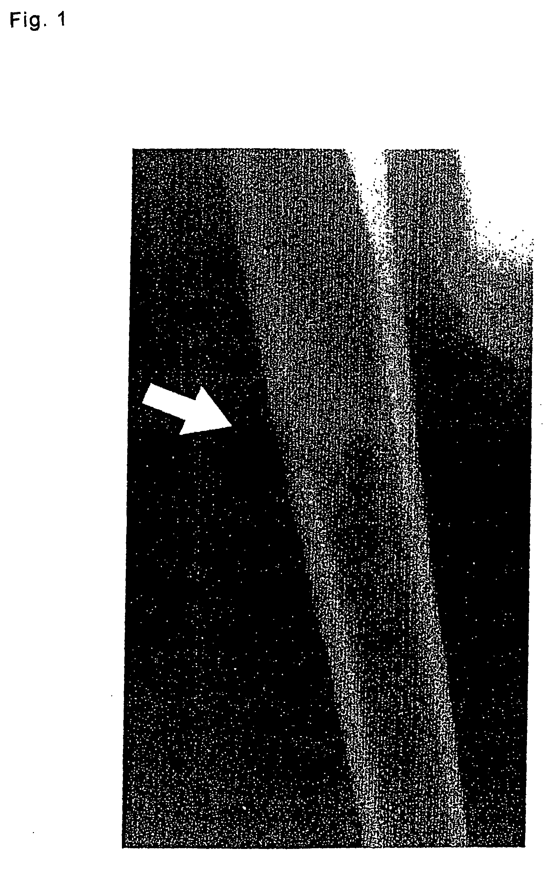

Figure 1 is a radiograph of periphery of a hole made in a tibia of a Japanese white rabbit 7 weeks after administration of a pasty mixture (control) containing a microcapsule without Compound A.

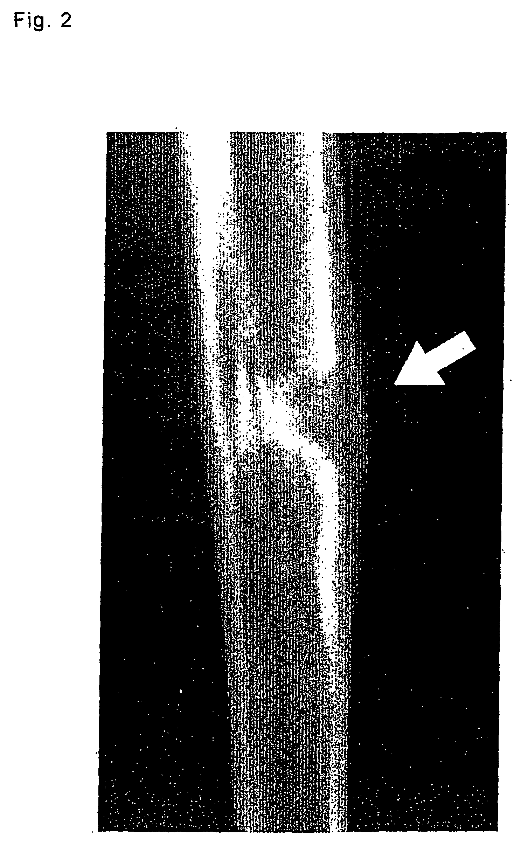

Figure 2 is a radiograph of a periphery of a hole made in a tibia of a Japanese white rabbit 7 weeks after administration of the sustained-release paste of the present invention containing a microcapsule of Compound A.

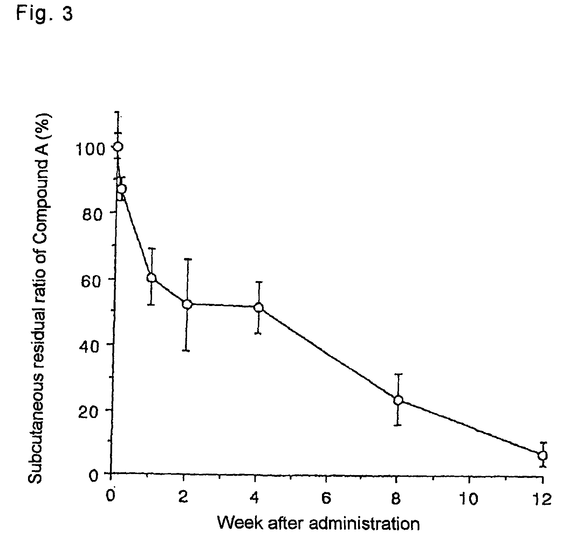

Figure 3 is a graph showing a change in the residual ratio of Compound A at an administration site after subcutaneous implantation of the sustained-release paste of the present invention containing a microcapsule of Compound A into the backs of rats. The X-axis represents a time after administration (week), and the Y-axis represents the subcutaneous residual ratio (%) of Compound A.

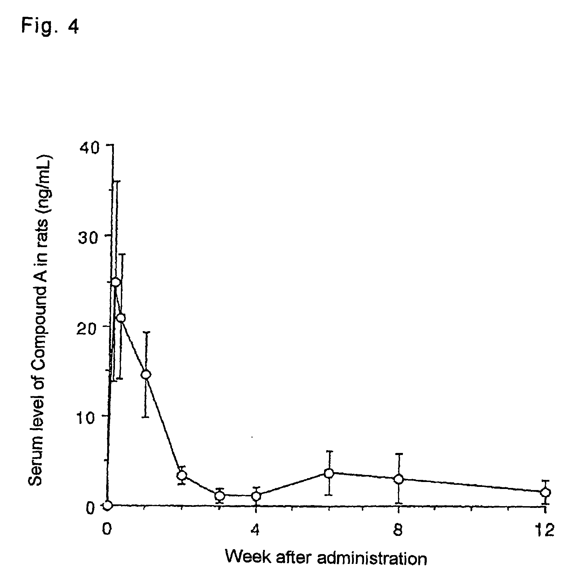

Figure 4 is a graph showing a change in the concentration of Compound A in the serum after subcutaneous implantation of the sustained-release paste of the present invention containing a microcapsule of Compound A into the backs of rats. The X-axis represents a time after administration (week), and the Y-axis represents the serum concentration (ng/ml) of Compound A.

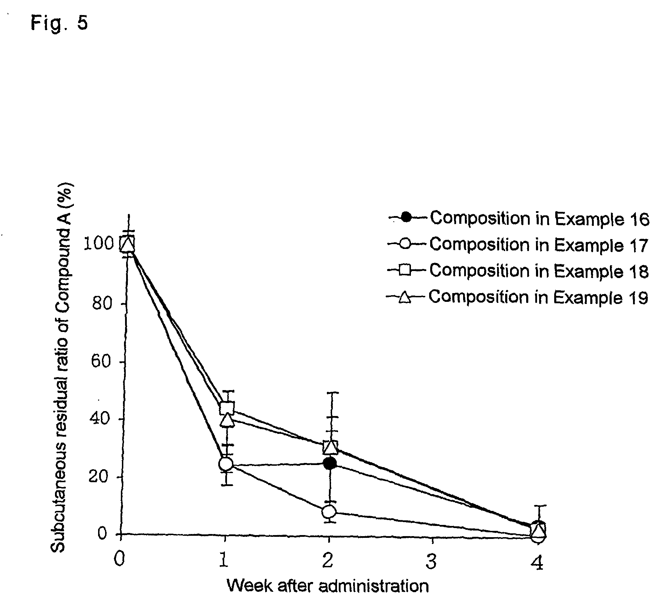

Figure 5 is a graph showing a change in the residual ratio of Compound A after subcutaneous implantation of the sustained-release pastes obtained in Examples 16 to 19 (0.14 ml) into the backs of rats. The X-axis represents a time after administration (week), and the Y-axis represents the subcutaneous residual ratio (%) of Compound A. The symbol-●- represents the subcutaneous residual ratio of Compound A in the sustained-release paste obtained in Example 16, the symbol -○- represents the subcutaneous residual ratio of Compound A in the sustained-release paste obtained in Example 17, the symbol -□- represents the subcutaneous residual ratio of Compound A in the sustained-release paste obtained in Example 18, and the symbol -Δ- represents the subcutaneous residual ratio of Compound A in the sustained-release paste obtained in Example 19, respectively.

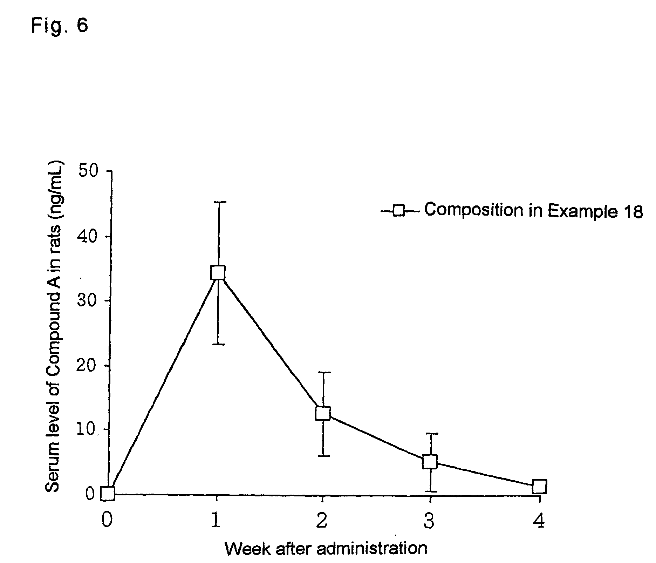

Figure 6 is a graph showing a change in the concentration of Compound A in the serum after subcutaneous implantation of the sustained-release paste obtained in Example 18 (0.14 ml) into the backs of rats. The X-axis represents a time after administration (week), and the Y-axis represents the serum concentration (ng/ml) of Compound A.

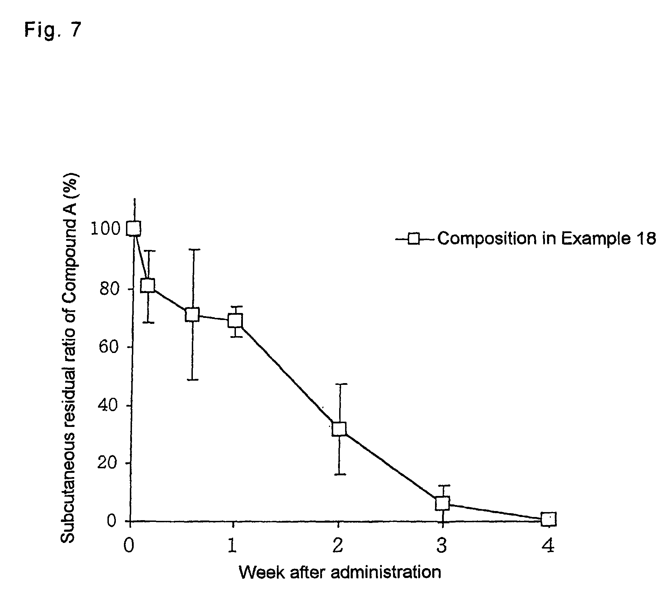

Figure 7 is graph showing a change in the residual ratio of Compound A in the serum after subcutaneous implantation of the sustained-release paste obtained in Example 18 (0.6 ml) into the backs of rats. The X-axis represents a time after administration (week), and the Y-axis represents the subcutaneous residual ratio (%) of Compound A.

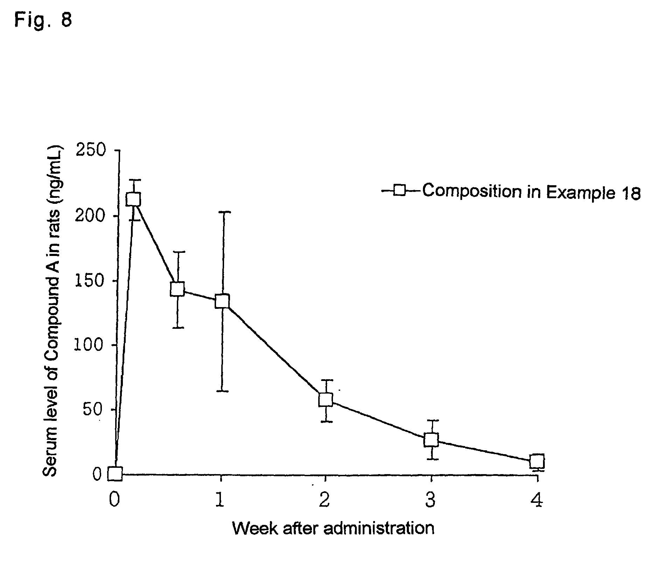

Figure 8 is a graph showing a change in the concentration of Compound A in the serum after subcutaneous implantation of the sustained-release paste obtained in Example 18 (0.6 ml) into the backs of rats. The X-axis represents a time after administration (week), and the Y-axis represents the serum concentration (ng/ml) of Compound A.

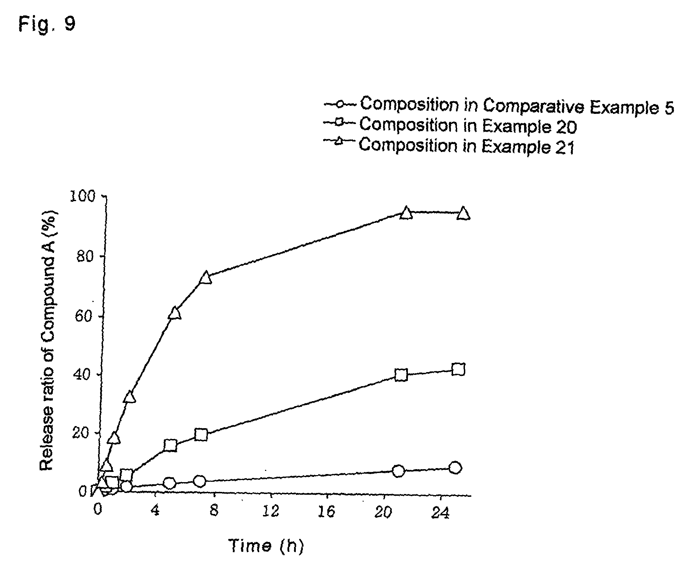

Figure 9 is a graph showing changes in the release ratio of Compound A from the mixture obtained in Comparative Example 5, the sustained-release pastes obtained in Examples 20 and 21 in an *in vitro* releasing test via a paddle method. The X-axis represents a time after incubation (hour), and the Y-axis represents the release ratio (%) of Compound A. The symbol -○- represents the release ratio of Compound A from the mixture obtained in Comparative Example 5, the symbol -□- represents the release ratio of Compound A from the sustained-release paste obtained in Example 20, and the symbol -Δ- represents the release ratio of Compound A from the sustained-release paste obtained in Example 21, respectively.

### The Best Mode for Carrying Out the Invention

As for the calcium component used in the present invention, any known calcium compound may be employed as long as they exhibit a hydraulic setting property at an ordinary temperature, including, for example, phosphate such as calcium dihydrogenphosphate, dicalcium phosphate anhydrate, dicalcium phosphate dihydrate, α-tricalcium phosphate, tetracalcium phosphate, sulfate such as calcium sulfate hemihydrate (e.g.,α-calcium sulfate hemihydrate, (β-calcium sulfate hemihydrate and the like), carbonate such as calcium carbonate and the like, used is a calcium component or any combination of two or more of calcium components showing hydraulic setting property, which is selected from the above cited calcium component. In particular, preferred are calcium dihydrogenphosphate, α-tricalcium phosphate, calcium sulfate hemihydrate (e.g., α-calcium sulfate hemihydrate, (β-calcium sulfate hemihydrate and the like), which by itself exhibits hydraulic setting property, and a combination of 2, or 3 or more components of calcium phosphate which can exhibits hydraulic setting property by combining them. Preferrerbly, a combination of calcium phosphate exhibiting a hydraulic setting property includes, for example, a combination of an anhydrate or dihydrate of dicalcium phosphate and tetracalcium phosphate, a combination of dicalcium phosphate dihydrate and calcium carbonate, and the like. Additionally, although α-tricalcium phosphate exhibits hydraulic setting property by itself, it is preferred to use it by combining with an anhydrate or a dihydrate of dicalcium phosphate or tetracalcium phosphate (e.g., a combination of an anhydrate or dihydrate of dicalcium phosphate and α-tricalcium phospahte, a combination of tetracalcium phosphate and α-tricalcium phosphate). As for the combination of calcium phosphate exhibiting hydraulic setting property, any combination may be employed without any limitation, as long as they change their structure to octacalcium phosphate or apatite *in vivo* during setting, but it is preferred that the molar ratio of calcium (Ca) and phosphorus (P) in the combination, Ca/P, is 1.1-1.8, and, particularly preferred is 1.2-1.67. In order to adjust the molar ratio of calcium and phosphorus in the combination within the above range, for example, phosphate such as sodium dihydrogenphosphate may be added to the combination.

In addition, as for the calcium component used in the present invention, a commercially available compound comprising mainly a calcium component (e.g., a powder part of Norian SRS (TRADE NAME, developed by Norian Corporation), a powder part of Biopex (TRADE NAME, supplied by Welfide Corporation)) may also be used.

When a hydrate is used as the above calcium component, by mixing the hydrate with water, the number of water molecules of the hydrate becomes the same as those for another hydrate having a different number of hydration water molecules or an anhydrate, but the calcium component in the paste of the present invention, as used herein, is defined based on the calcium component when it is incorporated before mixing with water.

The particle size of powder of a calcium component used in preparing a sustained-release paste of the present invention is not particularly limited, but the average particle size is preferably about 300 µm or less (e.g., about 0.1-300 µm), more preferably about 100 µm or less (e.g., about 0.1-100 µm), and most preferably about 50 µm or less (e.g., about 0.1-50 µm) in consideration of improving easiness in mixing to produce the sustained-release paste of the present invention.

Viscosity-increasing agent used in the present invention controls the setting rate of the calcium component exhibiting hydraulic setting property to imparting the sustained-release paste with spreadability, an application property, adherability and plasticity, and, furthermore, it also has a function to form a release channel for sustained-release of an osteogenesis promoter incorporated in the sustained-release paste of the present invention. Therefore, as the viscosity-increasing agent used in the present invention, any viscosity-increasing agent is not particularly limited as long as they are pharmacologically acceptable and capable of exhibiting the above function, but, for example, a biocompatible polymer, a sugar, a sugar alcohol, a polyalcohol and the like may be preferably used.

Additionally, a biocompatible polymer used in the sustained-release paste of the present invention is preferably a bioabsorbable polymer, and, in particular, a polymer which is water soluble and which does not form a insoluble salt by contacting with calcium is preferred. Such a biocompatible polymer includes, for example, a polysaccharide from a microorganisms such as dextran, sulfated dextran, pullulan; a polysaccharide such as gum arabic; a water-soluble cellulose derivative such as methylcellulose, carboxymethylcellulose and a salt thereof, hydroxypropylcellulose, hydroxypropylmethylcellulose; a starch and a derivative thereof such as starch, sodium carboxymethylstarch, hydroxyethylstarch, hydroxypropylstarch; chitin/chitosan and derivatives thereof such as chitin, chitosan; carboxymethylchitin; a mucopolysaccharide and a salt thereof such as hyaluronic acid and a salt thereof, chondoroitin sulfate and a salt thereof, dermatan sulfate and a salt thereof, heparan sulfate and a salt thereof, ketaran sulfate and a salt thereof; a water soluble protein such as gelatin, albumin; a water soluble synthetic polymer such as carboxy vinyl polymer, polypropylene glycol, polyethylene glycol, poly(vinyl alcohol), poly(vinyl pyrrolidone), poloxamer, polyamino acid and a salt thereof; glycogen and the like. Above all, dextran is particularly preferred.

Such a sugar/sugar alcohol includes, for example, glucose, sucrose, maltose, lactose, xylitol, mannitol, sorbitol and the like, and, xylitol, mannitol and sorbitol are particularly preferred. Further, such a polyalcohol includes, for example, glycol and glycerin.

In addition, xanthan gum, pectin, alginic acid and a salt thereof, collagen, fibrin and the like may be used as a viscosity-increasing agent in the sustained-release paste of the present invention.

The viscosity-increasing agent may be used alone or in combination of two or more of those selected from the above cited viscosity-increasing agents.

A form of the viscosity-increasing agent used in preparing the sustained-release paste of the present invention is not particularly limited, it may be solid powder or liquid at a room temperature. When the viscosity-increasing agent is solid powder, it may be used after pre-mixed with the above-mentioned calcium component homogeneously. On the other hand, when the viscosity-increasing agent is liquid at a room temperature, it may be used after dissolved or dispersed in the below-mentioned liquid component added to prepare the sustained-release paste of the present invention. In addition, the solid powder may be used after dissolved or suspended in the liquid component. Further, a part of the solid powder may be dissolved or suspended in the liquid component and the remaining powder may be mixed homogeneously with the calcium component as a solid powder.

In the present sustained-release paste, a duration for releasing an osteogenesis promoter which is an active component may be controlled by changing the amount of the viscosity-increasing agent to be added. Therefore, a compositional ratio of the viscosity-increasing agent to the calcium component can be selected from a wide rage depending on its type, setting of the releasing duration in interest. For example, when the releasing duration is from a few weeks to a few months, the compositional ratio of the viscosity-increasing agent is about 0.05-4, preferably about 0.1-2, more preferably about 0.15-1.5, further preferably about 0.2-1 parts by weight to a part of the calcium component.

Additionally, since the calcium component and the viscosity-increasing agent of the present invention are absorbed *in vivo* about 1 to 6 months after administration, an administration site with the paste is thoroughly replaced by a self bone.

In addition, the compositional ratio of the viscosity-increasing agent is about 1-500, preferably about 1-300, more preferably 1-100 parts by weight relative to a part of the osteogenesis promoter by weight. When the osteogenesis promoter is formulated into sustained-release preparations, the compositional ratio of the viscosity-increasing agent is about 0.1-50, preferably about 0.1-30, more preferably about 0.1-10 parts by weight relative to a part of the osteogenesis promoter by weight.

As an osteogenesis promoter, a peptide and a non-peptide substances are now known in the art, and both of them may be used in the sustained-release paste of the present invention. Examples of a non-peptide osteogenesis promoter include a sulfur-containing heterocyclic compound or a salt thereof described in JP-A 3-232880 (EP-A 376197), JP-A 4-364179 (EP-A 460488), JP-A 5-294960, JP-A 8-231569 (EP-A 719782) and JP-A2000-72678 (WO 00/09100) and the like; a benzopyran derivative or a salt thereof described in JP-A 7-291983 (corresponding to EP-A 625522); a phosphonic acid derivative or a salt thereof described in JP-A 8-73476 (WO 00/01267); a prostaglandin A1 derivative described in *Journal of Pharmacology Experimental Therapeutics,* vol. 258, pp. 1120-1126 (1991); a Vitamin D3 derivative described in *Bioorganic & Medicinal Chemistry Letters,* vol. 3, pp. 1815-1819 (1993); a benzylphosphonic acid derivative described in EP-A 524023; phosphonic acids described in *Bone,* vol. 13, pp. 249-255 (1992); Menatetrenone (produced by Eisai Co., Ltd.) and a Vitamin K2 derivatives described in Biochemical and Biophysical Research Communications, vol. 187, pp. 814-820 (1992), and the like.

In addition, examples of a peptide osteogenesis promoter include a bone morphogenetic protein (BMP), a firoblast growth factor (FGF), a platelet-derived growth factor (PDGF), a transforming growth factor (TGF-β), an insulin-like growth factor-1 and -2 (IGF-1 and -2), parathyroid hormone (PTH) and the like.

Among these osteogenesis promoter, a non-peptide osteogenesis promoter is preferred, for example, a derivative of benzothiopyran or benzothiepine. Examples of the derivatives of benzothiopyran of benzothiepine include compounds or a salt thereof described in JP-A 3-232880 (EP-A 376198), JP-A 4-364179 (EP-A 460788), JP-A 8-231569 (EP-A 719782) and JP-A 2000-72678 (WO 00/09100) and the like, and, among them, the compounds represented by the following formula (I): 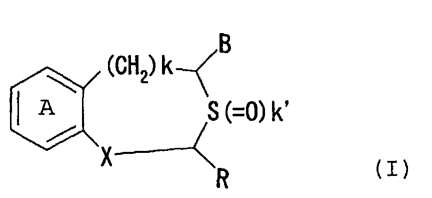 wherein the ring A is an optionally substituted benzene ring; R is a hydrogen atom or optionally substituted hydrocarbon group; B is an optionally esterified or amidated carboxy group; X is -CH(OH)- or -CO-; k is o or 1; and k' is 0, 1, or 2, or a salt thereof are preferred.

In the above-mentioned formula (I), the substituent on the substituted benzene ring represented by the ring A includes, for example, a halogen atom, a nitro group, an optionally substituted alkyl group, an optionally substituted hydroxy group, an optionally substituted mercapto group, an optionally substituted amino group, an acyl group, a mono- or di-alkoxyphosphoryl group, a phosphono group, an optionally substituted aryl group, an optionally substituted aralkyl group or an optionally substituted aromatic heterocyclic group; 1 to 4 of these substituents, preferably 1 or 2, which may be the same or different each other, may be substituted on the benzene ring.

The "halogen atom" includes, for example, fluorine, chlorine, bromine, iodine, and the like.

The alkyl group of the "optionally substituted alkyl group" includes, preferably, alkyl of 1 - 10 carbon atoms (e.g., methyl, ethyl, propyl, isopropyl, butyl, isobutyl, sec-butyl, tert-butyl, pentyl, isopentyl, neopentyl, hexyl, heptyl, octyl, nonyl, decyl, etc.), cycloalkyl of 3 - 7 carbon atoms (e.g., cyclopropyl, cyclobutyl, cyclohexyl, cycloheptyl, etc.), and the like; these may be substituted by 1 to 3 substituents such as, for example, halogen atom (e.g., fluorine, chlorine, bromine, iodine, etc.), hydroxy, alkoxy group of 1 - 6 carbon atoms (e.g., methoxy, ethoxy, propoxy, butoxy, hexyloxy, etc.), mono- or di-C₁₋₆ alkoxyphosphoryl group (e.g., methoxyphosphoryl, ethoxyphosphoryl, dimethoxyphosphoryl, diethoxyphosphoryl, etc.), phosphono, and the like.

The substituted alkyl group is exemplified by, for example, trifluoromethyl, trifluoroethyl, trichloromethyl, hydroxymethyl, 2-hydroxyethyl, methoxyethyl, 1-methoxyethyl, 2-methoxyethyl, 2,2-diethoxyethyl, 2-diethoxyphosphorylethyl, phosphonomethyl, and the like.

The substituted hydroxy group in the "optionally substituted hydroxy group" is exemplified by, for example, alkoxy group, alkenyloxy group, aralkyloxy group, acyloxy group, aryloxy group, and the like.

The "alkoxy group" includes, preferably, alkoxy group of 1-10 carbon atoms (e.g., methoxy, ethoxy, propoxy, butoxy, tert-butoxy, pentyloxy, hexyloxy, heptyloxy, nonyloxy, etc.), cycloalkoxy group of 4 - 6 carbon atoms (e.g., cyclobutoxy, cyclopentoxy, cyclohexyloxy, etc.). The "alkenyloxy group" includes, preferably, those of 2 - 10 carbon atoms, e.g., allyloxy, crotyloxy, 2-pentenyloxy, 3-hexenyloxy, 2-cyclopentenylmethoxy, 2-cyclohexenylmethoxy, etc. The "aralkyloxy group" includes, preferably, those of 6 - 19 carbon atoms, more preferably, (C₇-C₁₄) aryl- (C₁-C₄) alkyloxy (e.g., benzyloxy, phenethyloxy, etc.) . The "acyloxy group" includes, preferably, alkanoyloxy group, for example, those of 2 - 10 carbon atoms (e.g., acetyloxy, propionyloxy, *n*-butyryloxy, hexanoyloxy, etc.). The "aryloxy group" includes, preferably, those of 6 - 14 carbon atoms (e.g., phenoxy, biphenyloxy, etc.) . These groups may further be substituted by 1 - 3 of the same substituents as mentioned above such as, for example, halogen atom, hydroxy, alkoxy group of 1 - 6 carbon atoms, mono- or di-C₁₋₆ alkoxyphosphoryl group, phosphono group, and the like. The substituted hydroxy group is exemplified by, for example, trifluoromethoxy, 2,2,2-trifluoroethoxy, difluoromethoxy, 2-methoxyethoxy, 4-chlorobenzyloxy, 2-(3,4-dimethoxyphenyl) ethoxy, and the like.

The mercapto group in the "optionally substituted mercapto group" is exemplified by, for example, alkylthio group, aralkylthio group, acylthio group, and the like. The "alkylthio group" includes, preferably, alkylthio group of 1 - 10 carbon atoms (e.g., methylthio, ethylthio, propylthio, butylthio, pentylthio, hexylthio, heptylthio, nonylthio, etc.), cyclo-alkylthio group of 4 - 6 carbon atoms (e.g., cyclobutylthio, cyclopentylthio, cyclohexylthio, etc.), and the like. The "aralkylthio group" includes, preferably, those of 7 - 19 carbon atoms, more preferably, (C₆-C₁₄) aryl- (C₁-C₄)alkylthio group, e.g., benzylthio, phenethylthio, and the like. The "acylthio group" includes, preferably, alkanoylthio, for example, those of 2 - 10 carbon atoms (e.g., acetylthio, propionylthio, *n*-butyrylthio, hexanoylthio, etc.). These groups may further be substituted by 1 - 3 of the same substituents as mentioned above such as, for example, halogen atom, hydroxy, alkoxy group of 1 - 6 carbon atoms, mono- or di-C₁₋₆ alkoxyphosphoryl group, phosphono, and the like. The substituted mercapto group is exemplified by, for example, trifluoromethylthio, 2,2,2-trifluoroethylthio, 2-methoxyethylthio, 4-chlorobenzylthio, 3,4-dichlorobenzylthio, 4-fluorobenzylthio, 2-(3,4-dimethoxyphenyl)ethylthio, and the like.

The substituent of the substituted amino group in the "optionally substituted amino group" includes the same alkyl group of 1 - 10 carbon atoms as mentioned above, alkenyl group of 2 - 10 carbon atoms (e.g., allyl, vinyl, 2-penten-1-yl, 3-penten-1-yl, 2-hexen-1-yl, 3-hexen-1-yl, 2-cyclohexenyl, 2-cyclopentenyl, 2-methyl-2-propen-1-yl, 3-methyl-2-buten-1-yl, etc.), aryl group of 6 - 14 carbon atoms (e.g., phenyl, naphthyl, etc.), and aralkyl group of 7 - 19 carbon atoms (e.g., benzyl, phenethyl, etc.); and these may be used alone or in combination of two identical or different groups. These groups may be substituted by the same halogen atom, alkoxy group of 1 - 6 carbon atoms, mono- or di-C₁₋₆ alkoxyphosphoryl group, phosphono group, and the like, as mentioned above.

The substituted amino group is exemplified by, for example, methylamino, dimethylamino, ethylamino, diethylamino, dibutyl amino, diallylamino, cyclohexylamino, phenylamino or *N*-methyl-*N*-phenylamino, *N*-methyl-*N*-(4-chlorobenzyl)amino, *N,N*-di(2-methoxyethyl)amino, and the like.

As for the "acyl group", an organic carboxylic acyl group or sulfonic acyl group having a hydrocarbon group of 1 - 6 carbon atoms (e.g., C₁₋₆ alkyl (e.g., methyl, ethyl, *n*-propyl, hexyl, etc.), phenyl, etc.) may be used. As for the "organic carboxylic acyl group", for example, formyl, alkylcarbonyl group of 1 - 10 carbon atoms (e.g., acetyl, propionyl, butyryl, valeryl, pivaloyl, hexanoyl, octanoyl, cyclobutanecarbonyl, cyclo-hexanecarbonyl, cycloheptanecarbonyl, etc.), alkenylcarbonyl group of 2 - 10 carbon atoms (e.g., crotonyl, 2-cyclohexenecarbonyl, etc.), arylcarbonyl group of 6 - 14 carbon atoms (e.g., benzoyl, etc.), aralkylcarbonyl group of 7 - 19 carbon atoms (e.g., benzylcarbonyl, benzhydrylcarbonyl, etc.), 5- or 6-membered aromatic heterocyclic carbonyl group (e.g., nicotinoyl, 4-thiazolylcarbonyl, etc.), 5- or 6-membered aromatic heterocyclic acetyl group (e.g., 3-pyridylacetyl, 4-thiazolylacetyl, etc.), may be used. As for the "sulfonic acyl group having a hydrocarbon group of 1 - 6 carbon atoms", for example, methanesulfonyl, ethanesulfonyl, and the like may be used. These groups may be substituted by 1 - 3 of the same substituents as mentioned above, such as halogen atom, hydroxy, alkoxy group of 1 - 6 carbon atoms, amino, and the like. The acyl group is exemplified by, for example, trifluoroacetyl, trichloroacetyl, 4-methoxybutyryl, 3-cyclohexyloxypropionyl, 4-chlorobenzoyl, 3,4-dimethoxybenzoyl, and the like.

As for the "mono- or di-alkoxyphosphoryl group", for example, mono-C₁₋₆ alkoxyphosphoryl group such as methoxyphosphoryl, ethoxyphosphoryl, propoxyphosphoryl, isopropoxyphosphoryl, butoxyphosphoryl, pentyloxyphosphoryl, hexyloxyphosphoryl, and the like, and di-C₁₋₆ alkoxyphosphoryl group such as dimethoxyphosphoryl, diethoxyphosphoryl, dipropoxyphosphoryl, diisopropoxyphosphoryl, dibutoxyphosphoryl, dipentyloxyphosphoryl, dihexyloxyphosphoryl, and the like may be used. Preferably, di-C₁₋₆ alkoxyphosphoryl group, for example, dimethoxyphosphoryl, diethoxyphosphoryl, dipropoxyphosphoryl, diisopropoxyphosphoryl, ethylenedioxyphosphoryl, dibutoxyphosphoryl, and the like may be used.

As for the aryl group in the "optionally substituted aryl group", those of 6 - 14 carbon atoms, for example, phenyl, naphthyl, anthryl, and the like, may preferably be used. These groups may be substituted by 1 - 3 of the same substituents as mentioned above, such as alkyl group of 1 - 10 carbon atoms, halogen atom, hydroxy, alkoxy group of 1 - 6 carbon atoms, and the like. The substituted aryl group is exemplified by, for example, 4-chlorophenyl, 3,4-dimethoxyphenyl, 4-cyclohexylphenyl, 5,6,7,8-tetrahydro-2-naphthyl, and the like.

As for the aralkyl group in the "optionally substituted aralkyl group", those of 7 - 19 carbon atoms, for example, benzyl, naphthylethyl, trityl, and the like may preferably be used. These groups may be substituted on their aromatic ring by 1 - 3 of the same substituents as mentioned above such as alkyl group of 1 - 10 carbon atoms, halogen atom, hydroxy, alkoxy group of 1 - 6 carbon atoms, and the like. The substituted aralkyl group is exemplified by, for example, 4-chlorobenzyl, 3,4-dimethoxybenzyl, 4-cyclohexylbenzyl, 5,6,7,8-tetrahydro-2-naphthylethyl, and the like.

As for the aromatic heterocyclic group in the "optionally substituted aromatic heterocyclic group", 5- or 6-membered ones having 1 to 4 of nitrogen atom, oxygen atom and/or sulfur atom, for example, furyl, thienyl, imidazolyl, thiazolyl, oxazolyl, thidiazolyl, and the like, may preferably be used. These groups may be substituted by 1- 3 of the same substituents as mentioned above such as alkyl group of 1 - 10 carbon atoms, halogen atom, hydroxy, alkoxy group of 1 - 6 carbon atoms, and the like.

When two alkyl groups are placed adjacent to each other on the benzene ring A, they may be bound to each other to form an alkylene group of the formula: -(CH₂)ₘ- where m is an integer of 3 - 5 (e.g., trimethylene, tetramethylene, pentamethylene, etc.), and when two alkoxy groups are placed adjacent to each other, they may form an alkylenedioxy group of the formula: -O- (CH₂)ₙ-O- where n is an integer of 1 - 3 (e.g., methylenedioxy, ethylenedioxy, trimethylenedioxy, etc.). In such a case, a 5-to 7-membered ring is formed together with the carbon atoms on the benzene ring.

In the above-mentioned formula (I), R represents a hydrogen atom or optionally substituted hydrocarbon group.

As for the hydrocarbon group in the "optionally substituted hydrocarbon group" represented by R, an alkyl group (preferably, alkyl group of 1 - 10 carbon atoms, including, e.g., methyl, ethyl, propyl, isopropyl, butyl, isobutyl, *sec*-butyl, *tert*-butyl, pentyl, isopentyl, neopentyl, hexyl, etc.), the same alkenyl group as mentioned above (preferably, alkenyl group of 2 - 10 carbon atoms), as well as the same aryl group (preferably, aryl group of 6 - 14 carbon atoms), aralkyl group (preferably, aralkyl group of 7 - 19 carbon atoms), and the like as mentioned above may be used. As for the substituent on the hydrocarbon group, a 5- or 6-membered aromatic heterocylic group (e.g., furyl, thienyl, imidazolyl, thiazolyl, oxazolyl, thiadiazolyl, etc.), the same halogen atom as mentioned above, the same di-C₁₋₆ alkoxyphosphoryl group as mentioned above, phosphono, and the like may be used.

In the above-mentioned formula (I), B is an optionally esterified or amidated carboxyl group.

As for the esterified carboxyl group of the "optionally esterified carboxyl group" represented by B, for example, an alkoxycarbonyl group, preferably, C₁₋₁₀ alkoxycarbonyl group (e.g., methoxycarbonyl, ethoxycarbonyl, propoxycarbonyl, butoxycaronyl, etc.), aryloxycarbonyl group, preferably, C₆₋₁₄ aryloxycarbonyl group (e.g., phenoxycarbonyl, etc.), aralkyloxycarbonyl group, preferably, C₇₋₁₉ aralkyloxycarbonyl group (e.g., benzyloxycarbonyl, etc.), and the like maybe used.

The amidated carboxyl group of the "optionally amidated carboxyl group" represented by B includes optionally substituted carbamoyl groups represented by the formula: CON(R¹) (R²) wherein R¹ and R² each is a hydrogen atom, optionally substituted hydrocarbon group or optionally substituted 5 - 7-membered heterocyclic group.

As for the hydrocarbon group in the "optionally substituted hydrocarbon group" represented by R¹ and R², an alkyl group, preferably, alkyl group of 1 - 10 carbon atoms (e.g., methyl, ethyl, propyl, isopropyl, butyl, isobutyl, *sec*-butyl, *tert*-butyl, pentyl, isopentyl, neopentyl, hexyl, heptyl, octyl, nonyl, decyl, etc.), alkenyl group, preferably, alkenyl group of 2 - 10 carbon atoms (e.g., allyl, vinyl, 2-penten-1-yl, 3-penten-1-yl, 2-hexen-1-yl, 3-hexen-1-yl, 2-cyclohexenyl, 2-cyclopentenyl, 2-methyl-2-propen-1-yl, 3-methyl-2-buten-1-yl, etc.), aryl group, preferably, aryl group of 6 - 14 carbon atoms (e.g., phenyl, naphthyl, anthryl, etc.), aralkyl group, preferably, aralkyl group of 7 - 19 carbon atoms (e.g., benzyl, naphthylethyl, trityl, etc.), and the like may be used. These hydrocarbon groups may be substituted by 1 - 3 substituents such as (i) a halogen atom (e.g., fluorine, chlorine, bromine, iodine, etc.), (ii) a hydroxy group, (iii) an alkoxy group of 1 - 6 carbon atoms (e.g., methoxy, ethoxy, propoxy, butoxy, *tert*-butoxy, pentyloxy, hexyloxy, etc.), (iv) an amino group optionally substituted by alkyl group of 1 - 6 carbon atoms (e.g., methyl, ethyl, propyl, isopropyl, butyl, isobutyl, *sec*-butyl, *tert*-butyl, pentyl, isopentyl, neopentyl, hexyl, etc.) (e.g., amino, methylamino, ethylamino, dimethylamino, diethylamino, dipropylamino, etc.), (v) an amino group substituted by an acyl group (e.g., alkanoyl group of 1- 10 carbons, etc.) (e.g., acetylamino, propionylamino, benzoylamino, etc.), (vi) a carbamoyl group optionally substituted by alkyl group of 1 - 6 carbon atoms (e.g., carbamoyl, methylcarbamoyl, dimethylcarbamoyl, diethylcarbamoyl, etc.), (vii) a C₁₋₆ alkoxycarbonyl group (e.g., methoxycarbonyl, ethoxycarbonyl, propoxycarbonyl, etc.), (viii) a mono- or di-alkoxyphosphoryl group (e.g., mono- or di-C₁₋₆ alkoxyphosphoryl group (e.g., dimethoxyphosphoryl, diethoxyphosphoryl, ethylenedioxyphosphoryl, etc.), etc.), (ix) a mono- or dialkoxyphosphorylalkyl group (e.g., mono- or di-C₁₋₆ alkoxyphosphoryl-C₁₋₃ alkyl group (e.g., methoxyphosphorylmethyl, ethoxyphosphorylmethyl, methoxyphosphorylethyl, ethoxyphosphorylethyl, dimethoxyphosphorylmethyl, diethoxyphosphorylmethyl, dimethoxyphosphorylethyl, diethoxyphosphorylethyl, etc.), etc.), (x) a group of the formula: 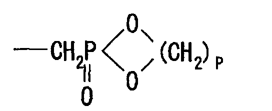 wherein p is an integer of 2 to 4,
(xi) a phosphono group, (xii) an aromatic heterocyclic group (the same as defined above), and the like.

As for the 5- to 7-membered heterocyclic group in the "optionally substituted 5- to 7-membered heterocyclic group" represented by R¹ and R², for example, a 5- to 7-membered heterocyclic group containing one sulfur atom, nitrogen atom or oxygen atom, 5- or 6-membered heterocyclic group containing 2 to 4 nitrogen atoms, or 5- or 6-membered heterocyclic group containing 1 or 2 nitrogen atoms and one sulfur atom or oxygen atom may be used. These heterocyclic groups may be condensed with a 6-membered ring containing up to 2 nitrogen atoms, benzene ring, or 5-membered ring containing one sulfur atom. As for the substituent which may be substituted on the "optionally substituted 5- to 7-membered heterocyclic group", 1 to 4 of the same substituents as those which may be substituted on the hydrocarbon group of the "optionally substituted hydrocarbon group" represented by the above-mentioned R¹ and R², may be used.

Preferred example of the 5- to 7-membered heterocyclic group represented by R¹ and R² includes, for example, 2-pyridyl, pyrimidyl, pyrazinyl, pyridazinyl, pyrazolyl, imidazolyl, thiazolyl, oxazolyl, tetrazolyl, thiadiazolyl, oxadiazolyl, triazinyl, triazolyl, thienyl, pyrrolyl, pyrrolinyl, furyl, pyrrolidinyl, benzo-thienyl, indolyl, imidazolidinyl, piperidyl, piperidino, piperadinyl, morpholinyl, morpholino, pyrido[2,3-d]pyrimidyl, benzopyranyl, 1,8-naphthylidyl, quinolyl, thieno[2,3-b]-pyridyl, and the like.

R¹ and R² in -N(R¹) (R²) taken together may form a 5- to 7-membered ring which includes, for example, morpholine, thiomorpholine, piperidine, homopiperazine, piperazine, pyrrolidine, pyrroline, pyrazoline, imidazoline, imidazolidine, thiazolidine, azepine, and the like.

The preferred substituted alkyl group in the "optionally substituted hydrocarbon group" represented by R¹ and R² is exemplified by, for example, trifluoromethyl, trifluoroethyl, difluoromethyl, trichloromethyl, 2-hydroxyethyl, 2-methoxyethyl, 2-ethoxyethyl, 2,2-dimethoxyethyl, 2,2-diethoxyethyl, 2-pyridylmethyl, 3-pyridylmethyl, 4-pyridylmethyl, 2-(2-thienyl) ethyl, 3-(3-furyl)propyl, 2-morpholinoethyl, 3-pyrrolylbutyl, 2-piperidinoethyl, 2-(*N, N*-dimethylamino)ethyl, 2-(*N*-methyl-*N*-ethylamino)ethyl, *2- (N, N*-diisopropylamino) ethyl, 5- (*N*,*N*-dimethylamino) pentyl, *N,N*-dimethylcarbamoylethyl, *N,N*-dimethylcarbamoylpentyl, ethoxycarbonylmethyl, isopropoxycarbonylethyl, *tert*butoxycarbonylpropyl, 2-diethoxyphosphorylethyl, 3-dipropoxyphosphorylpropyl, 4-dibutoxyphosphorylbutyl, ethylenedioxyphosphorylmethyl, 2-phosphonoethyl, 3-phosphonopropyl, and the like. The substituted aralkyl group is exemplified by, for example, 4-chlorobenzyl, 3-(2-fluorophenyl)propyl, 3-methoxybenzyl, 3,4-dimethoxyphenethyl, 4-ethylbenzyl, 4-(3-trifluoromethylphenyl)butyl, 4-acetylaminobenzyl, 4-dimethylaminophenethyl, 4-diethoxyphosphorylbenzyl, 2-(4-dipropoxyphosphorylmethylphenyl)ethyl, and the like. The substituted aryl group is exemplified by, for example, 4-chlorophenyl, 4-cyclohexylphenyl, 5,6,7, 8-tetrahydro-2-naphthyl, 3-trifluoromethylphenyl, 4-hydroxyphenyl, 3,4,5-trimethoxyphenyl, 6-methoxy-2-naphthyl, 4-(4-chlorobenzyloxy)phenyl, 3,4-methylenedioxyphenyl, 4-(2,2,2-trifluoroethoxy)phenyl, 4-propionylphenyl, 4-cyclohexanecarbonylphenyl, 4-dimethylaminophenyl, 4-benzoylaminophenyl, 4-diethoxycarbamoylphenyl, 4-*tert*butoxycarbonylphenyl, 4-diethoxyphosphorylphenyl, 4-diethoxyphosphorylmethylphenyl, 4-(2-diethoxyphosphorylethyl)phenyl, 2-diethoxyphosphorylmethylphenyl, 3-diethoxyphosphorylmethylphenyl, 4-dipropoxyphosphorylphenyl, 4-(2-phosphonoethyl) phenyl, 4-phosphonomethylphenyl, 4-phosphonophenyl, and the like. The substituted 5- to 7-membered heterocyclic group is exemplified by, for example, 5-chloro-2-pyridyl, 3-methoxy-2-pyridyl, 5-methyl-2-benzothiazolyl, 5-methyl-4-phenyl-2-thiazolyl, 3-phenyl-5-isoxazolyl, 4-(4-chlorophenyl)-5-methyl-2-oxazolyl, 3-phenyl-1,2,4-thiadiazol-5-yl, 5-methyl-1,3,4-thiadiazol-2-yl, 5-acetylamino-2-primidyl, 3-methyl-2-thienyl, 4,5-dimethyl-2-furanyl, 4-methyl-2-morpholinyl, and the like.

In the above-mentioned groups, the ring A is a benzene ring optionally substituted by 1 or more, preferably 1 or 2 of the same or different substituents, such as halogen atom, optionally substituted alkyl group, optionally substituted hydroxy group, optionally substituted mercapto group and/or optionally substituted amino group.

Particularly preferred ring A is a benzene ring that may be substituted by 1 or 2 of substituents, such as halogen atom, alkyl group of 1 - 10 carbon atoms (more preferably, of 1 - 5 carbon atoms), alkoxy group of 1 - 10 carbon atoms (more preferably, of 1 - 5 carbon atoms), alkylenedioxy group of the formula: -O-(CH₂)ₙ-O- where n is an integer of 1 - 3 and/or alkylthio group of 1 - 10 carbon atoms (more preferably, of 1 - 5 carbon atoms).

Especially preferred example of ring A is a benzene ring that is substituted at the adjacent carbon atoms by an alkylenedioxy group of the formula: -O-(CH₂)ₙ-O- where n is an integer of 1 - 3.

R is preferably a hydrogen atom, C₁₋₆ alkyl group (e.g., methyl, ethyl, etc.) or phenyl.

B is preferably, for example, an alkoxycarbonyl group or a group of the formula: -CON(R¹) (R²) wherein R¹ and R² each is a hydrogen atom, optionally substituted hydrocarbon group or optionally substituted 5- to 7-membered heterocyclic group.

In a preferred example of R¹and R², R¹ is a hydrogen atom or alkyl group of 1 - 10 carbon atoms (e.g., methyl, ethyl, propyl, etc.), and R² is a phenyl or phenyl-C₁₋₃ alkyl group which may be substituted by halogen (e.g., fluorine, chlorine, bromine, etc.), C₁₋₆ alkoxy (e.g., methoxy, ethoxy, etc.), mono- or di-alkoxyphosphoryl (e.g., mono- or di-C₁₋₆ alkoxyphosphoryl such as dimethoxyphosphoryl, diethoxyphosphoryl, etc.), mono-or di-alkoxyphosphorylalkyl (e.g., mono- or di-C₁₋₆ alkoxyphosphoryl-C₁₋₆ alkyl such as dimethoxyphosphorylmethyl, diethoxyphosphorylmethyl, etc.) (the dialkyl in the di-C₁₋₆ alkoxy taken together may form a C₁₋₆ alkylene group) or C₁₋₆ alkoxycarbonyl (e.g., methoxycarbonyl, ethoxycarbonyl, etc.), or an optionally phenyl-substituted 5- or 6-membered heterocyclic group containing 1 or 2 nitrogen atoms or 1 nitrogen atom and 1 sulfur atom (e.g., pyridyl, etc.).

In an especially preferred example of R¹ and R², R¹ is a hydrogen atom, and R² is a phenyl group substituted by mono-or di-C₁₋₆ alkoxyphosphoryl-C₁₋₃ alkyl (e.g., 4-diethoxyphosphorylmethylphenyl, etc.).

In the above-mentioned formula (I), X is -CH (OH) - or -CO-, preferably, -CO-.

In the above-mentioned formula (I), k is 0 or 1, k' is 0, 1 or 2, and preferably, k is 1 and k' is 0.

In the above-mentioned formula (I), particularly preferred is, for example, an optically active benzothiepine derivative of the formula (II): 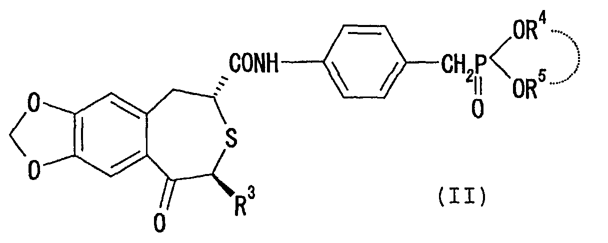 wherein R³ is C₁₋₆ alkyl group; R⁴ and R⁵ each is a C₁₋₆ alkyl group, or they taken together form a C₁₋₆ alkylene group.

In the above-mentioned formula (II), the "C₁₋₆ alkyl group" represented by R³, R⁴ and R⁵ includes methyl, ethyl, propyl, 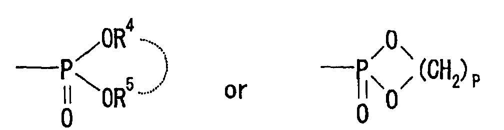 isopropyl, butyl, isobutyl, *sec*-butyl, *tert*-butyl, pentyl, isopentyl, neopentyl, hexyl, and the like. Preferably, an alkyl group of 1 to 4 carbon atoms is exemplified. R⁴ and R⁵ taken together may form a C₁₋₆ alkylene group, which may be represented by the formula:
wherein p is an integer of 2 to 4.

As for R³, R⁴ and R⁵, an alkyl group of 1 to 4 carbon atoms such as methyl, ethyl, etc. is preferred.

The compounds (II) are optically active isomers with a (2*R*,4*S*) configuration substantially containing no compound of (2*S*,4*R*) configuration, and it is better when the optical purity is nearer to 100%.

Particularly preferred one of the compounds (II) is, for example, (2*R*,4*S*)- (-)-*N*-[4- (diethoxyphosphorylmethyl)phenyl]-1,2,4,5-tetrahydro-4-methyl-7,8-methylenedioxy-5-oxo-3-benzothiepine-2-carboxamide (hereinafter sometimes referred to as Compound A) or a salt thereof. The structural formula of Compound A can be represented by the formula:

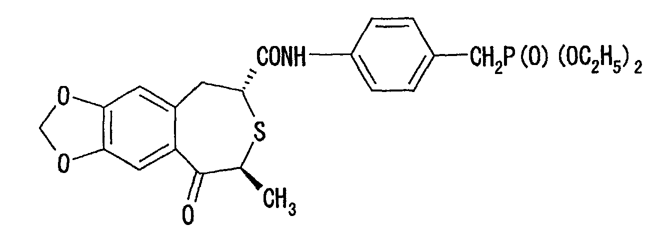

The benzothiopyran or benzothiepin derivatives used in the compositions of the invention, preferably the salts of benzothiopyran or benzothiepin derivatives having an osteogenesis or chondrogenesis promoting effect are, preferably, used as pharmacologically acceptable salts. The pharmacologically acceptable salts used include those with inorganic bases, organic bases, inorganic acids or organic acids, or with basic or acidic amino acids. The base that can form salts with the benzothiopyran or benzothiepin derivatives includes inorganic bases such as alkali metal (e.g., sodium, potassium, etc.), alkaline earth metal (e.g., calcium, magnesium, etc.), and organic bases such as trimethylamine, triethylamine, pyridine, picoline, *N,N*-dibenzylethylenediamine, diethanolamine, etc. The acid includes inorganic acids such as hydrochloric acid, hydrobromic acid, hydroiodic acid, phosphoric acid, nitric acid, sulfuric acid, etc., and organic acids such as formic acid, acetic acid, trifluoroacetic acid, oxalic acid, tartaric acid, fumaric acid, maleic acid, methanesulfonic acid, benzenesulfonic acid, *p*-toluenesulfonic acid, citric acid, etc. The basic or acidic amino acid includes, for example, arginine, lysine, aspartic acid, glutamic acid, etc.

Additionally, the benzothiopyran or benzothiepin derivatives or a salt thereof used in the present invention may be produced, for example, in the same process as described in JP-A 3-232880 (EP-A 376197), JP-A 4-364179 (EP-A 460488), or JP-A 8-231569 (EP-A 719782), or a process similar thereto or their equivalent process.

In the osteogenesis promoter used in the present invention, as a compound having a potent action, particularly preferred are (2*R*,4*S*)-(-)-*N*-[4-(diethoxyphosphorylmethyl)phenyl]-1,2,4,5-tetrahydro-4-methyl-7,8-methylenedioxy-5-oxo-3-benzothiepin-2-carboxamide (hereinafter, sometimes, referred to as "Compound A") and a salt thereof, and *N*-(4-dimethoxyphospholylmethylphenyl)-4-oxo-4*H*-1-benzopyran-2-carboxamide and a salt thereof disclosed in JP-A 8-231569 (EP-A 719782), diethyl 4-(7-cyclohexyl-3,4-dihydro-2-naphthalenecarboxamide)benzylphosphonate and a salt thereof, and the like. Among them, (2*R*, 4*S*)-(-)-*N*-[4-(diethoxyphosphorylmethyl)phenyl]-1,2,4,5-tetrahydro-4-methyl-7,8-methylenedioxy-5-oxo-3-benzothiepin-2-carboxamide (hereinafter, sometimes, referred to as "Compound A") and a salt thereof are particularly preferred.

The osteogenesis promoter used in the present invention includes both a material with osteoconductivity and a material with osteoinductivity.

An osteogenesis promoter may be used as it is in the form of a crystalline powder or an amorphous powder to incorporate in the sustained-release paste of the present invention. In addition, although the above-described compounds may be used as an osteogenesis promoter, as they are, or as salts thereof, they may also be used to incorporate in the sustained-release paste of the present invention after they are previously formulated into a sustained-release preparation. By formulating the osteogenesis promoter into a sustained-release preparation, an effect of release control can be imparting to the sustained-release preparation itself in addition to controlling the duration for releasing the osteogenesis promoter by changing the compositional ratio of the viscosity-increasing agent, as described above. Therefore, depending on a desired duration for releasing the osteogenesis promoter, the amount of the viscosity-increasing agent, as well as the composition or the amount of the sustained-release preparation incorporated in the paste can be appropriately varied.

When a non-peptide osteogenesis promoter is formulated into a sustained-release preparation, it is preferred to prepare a microcapsule or microsphere of it with a biodegradable polymer, as above described in, for example, JP-A 9-263545 (WO 96/39134). (See the Reference Example below.)

Since the sustained-release paste of the present invention continues setting after applied to a local site in the body, it shows a high retention property at the local site. At the same time, the set paste is gradually degraded at the retention site, gradually releasing the osteogenesis promoter. When a sustained-release preparation of an osteogenesis promoter is used, since the preparation itself exhibits a sustained-release effect, the duration for releasing the osteogenesis promoter at the local site can be further prolonged.

In the sustained-release paste of the present invention, although the compositional ratio of the osteogenesis promoter to the calcium component is not particularly limited, for example, the calcium component may be incorporated in about 1-500 parts by weight, preferably about 1-300 parts by weight, more preferably about 1-100 parts by weight, and most preferably about 2-100 parts by weight relative to a part of the osteogenesis promoter by weight.

The form of the osteogenesis promoter or a sustained-release preparation thereof used in the present invention is not particularly limited, and it may be either in a solid powder form or a liquid form at a room temperature. In the case of a solid powder form, it may be used after premixed homogeneously with the above calcium component. On the other hand, in the case of a liquid form at a room temperature, it may be used after dissolved or suspended in the below-described liquid component added in order to prepare the sustained-release paste of the present invention. In addition, the solid powder may also be used after dissolved or suspended in the liquid component.

The sustained-release paste of the present invention may be prepared by adding a liquid component consisting of water to a composition containing three components, the calcium component, the viscosity-increasing agent and the osteogenesis promoter, as described above, and by mixing them. At this point, for example, when all of the above three components are in a solid powder form, a liquid component is added to the homogeneous premixture of three components, which is, in turn, mixed until the liquid component and the solid components have been dispersed homogeneously. In addition, when the viscosity-increasing agent and the osteogenesis promoter are dissolved or suspended in the liquid component, the liquid component is added to the calcium component, and, then, they are mixed to disperse them homogeneously. When the viscosity-increasing agent is dissolved or suspended in the liquid component, the liquid component is added to the composition containing the calcium component and the osteogenesis promoter, and, then, they are mixed to disperse them homogeneously. When the osteogenesis promoter is dissolved in the liquid component, the liquid component is added to the composition containing the calcium component and the viscosity-increasing agent, and, then, they are mixed to disperse them homogeneously.

The time for mixing is varied depending on the types and amount of the calcium component and the viscosity-increasing agent, and the composition of the liquid component as discussed below, generally a few 10 seconds to about 5 minutes.

The mixed sustained-release paste of the present invention begins to set, but the setting rate is much slower than that for a so-called self-setting cement used as an artificial bone or a bone filling material. The setting time of the mixed sustained-release paste of the present invention is, specifically 1 hour or longer, more specifically 1 to 24 hours and, most specifically 1 to 18 hours (in particular, 3 to 12 hours). The setting time as used herein is measured by a Vicat indentation test according to a measurement method for a dental Zinc phosphate cement (JIS T6602). Specifically, the mixture is transported to a thermostatic chamber at 37 °C at about 100 % of a relative humidity 3 minutes after the start of mixing. Then, a 300 g of Vicat needle is gently dropped onto the surface of the mixture to check whether a needle mark is made. A time from the start of mixing to a time where a needle mark is not made any more is defined as a setting time.

Furthermore, the sustained-release paste of the present invention can retain plasticity a few hours after mixing. Specifically, the sustained-release paste of the present invention shows a compressive strength of 1.5 MPa or less (preferably, 1 MPa or less) even 14 hours after mixing. (See the Reference Example below.) The sustained-release paste of the present invention shows a compressive strength of, more specifically 0.05 to 1.5 MPa, most specifically 0.1 to 1 MPa even 14 hours after mixing. The sustained-release paste of the present invention shows a compressive strength of, specifically 3 MPa or less, more specifically 0.1 to 3 MPa, most specifically 0.2 to 2 MPa two weeks after mixing. The compressive strength is obtained by measuring a strength of set compositions (formed into a cylinder with a diameter of 1.0 cm and a height of 1.0 cm) using a universal testing machine (INTESCO Model 205E) after a predetermined period after mixing under a condition at 37 °C and a relative humidity of about 100 %.

The diametral tensile strength (DTS) 14 hours after mixing of the sustained-release paste of the present invention is specifically 0.4 MPa or less, more specifically 0.01 to 0.4 MPa, and most specifically 0.02 to 0.2 MPa. The diametral tensile strength (DTS) is measured using a set composition formed into a cylinder with a diameter of 1.0 cm and a height of 0.5 cm under the same condition for the compressive strength.

The adhesion force of the sustained-release paste of the present invention is specifically 6 Newton (N) or above, more specifically 6 to 50 N, most specifically 8 to 30 N. The adhesion force is obtained by putting a 0.1 ml of the mixture within two glass plates 5 minutes after mixing and by measuring a strength to peel the two glass plates 1 minute after holding the pressure at 29.4 N using the above universal testing machine (INTESCO Model 205E).

Since the sustained-release paste of the present invention has the above characteristic property, the paste can be clearly distinguished from the conventional artificial bone or bone filling material. In addition, on the basis of the above characteristic property, the present paste can be prepared prior to a surgical treatment such as a fracture operation, and the paste exhibits a characteristic effect to apply easily to a bone defect site a few minutes to a few hours after mixing it.

As a liquid component used in preparing the sustained-release paste of the present invention, for example, distilled water for injection may be used as it is, and, a physiological saline, a phosphate buffer, an aqueous solution of an inorganic acid or an organic acid or salts thereof may be also used.

For example, when the calcium component is phosphate, the setting rate can be increased by using a solution of an inorganic or an organic acid or salts thereof as a liquid component, as required. It is advantageous to increase the setting rate of the sustained-release paste of the present invention in the case where it is preferred to apply a paste having a certain hardness, for example, when a hemorrhage site and an adjacent region thereof are treated. The type of the inorganic or organic acid or the salts thereof usable in the aqueous solution is not particularly limited, phosphoric acid, acetic acid, lactic acid, citric acid, succinic acid, malic acid, malonic acid, gultaric acid, glycolic acid, tartaric acid and the like, or a sodium salt of lactic acid or succinic acid and the like are preferred. The concentration of these acids or salts thereof may be appropriately selected in a range non-toxic to a living body.

In addition, when the calcium component is phosphate, the setting rate can also be increased by a phosphate buffer having a almost neutral pH. The concentration of the phosphate buffer is not particularly limited, but is appropriately selected according to a period of time to apply to the living body from a time when preparing the sustained-release paste of the present invention. For example, it is in a range of about 0.1 M to 1 M.

Furthermore, when the calcium component is a sulfate, a preferred setting rate can be obtained by using distilled water, a saline or a phosphate buffered saline.

Volume ratio (g/ml) of liquid to the total amount of the osteogenesis promoter, the calcium component and the viscosity-increasing agent incorporated in the sustained-release paste of the present invention may be optionally varied depending on the purpose, for example, about 0.5-15 (g/ml), preferably about 1-10 (g/ml), more preferably about 1.5-7 (g/ml), and most preferably about 1.5-6 (g/ml).

In order to adjust the setting rate and the hardness of the sustained-release paste (such as the setting time, compressive strength, and diametral tensile strength), calcium sulfate dihydrate, apatite (e.g., hydroxyapatite, apatite carbonate, fluoroapatite), octacalcium phosphate, amorphous calcium phosphate and the like, which are excellent in osteoconductivity and bone affinity, may be incorporated in the calcium component of the present invention (e.g., calcium sulfate hemihydrate or a combination of calcium phosphates exhibiting a setting property, and the like). Their amount to be added may be optionally selected depending or the desired hardness or the desired setting time, for example, about 0.001 to 10 parts by weight, preferably about 0.001 to 7 parts by weight, more preferably about 0.001 to 5 parts by weight relative to a part of the calcium component by weight. Among the combination mentioned above, a combination of calcium sulfate hemihydrate and calcium sulfate dihydrate as a calcium component is particularly preferred.

When it is needed to promote a release of the osteogenesis promoter from the sustained-release paste of the present invention, water-soluble salts such as sodium chloride, sodium hydrogencarbonate, sodium carbonate may also be added other than the above essential components. The amount of these additives to be added is about 0.005-3 parts by weight, preferably about 0.01-1 parts by weight relative to a part of the calcium component by weight.

Since the sustained-release paste of the present invention can release gradually an osteogenesis promoter constantly for a long period, a constant potency can be obtained, and since it has affinity and adherability to a bone and an excellent osteoconductivity, it can be applied in treatment of a fracture or a bone defect, or in treatment of many types of bone diseases as a bone coaptation material in a bone transplantation. Further, in the regimen of an oral surgery, it can be applied for treating bone diseases such as palingenesis of a lost alveolus or increase in the amount of an alveolus, filling to a defect part in a palate.

The sustained-release paste of the present invention can be used in treating bone diseases such as a simple fracture, insufficient coaptation, a re-fracture, a bone defect, and the like. In addition, the sustained-release paste of the present invention can be used as a bone defect treating agent for osteogenesis promotion after abrating a focus of an osteomyelitis, a bone/joint infectious disease, a chronic joint disease, a metabolic bone disease, a traumatic bone disease or an osteosarcoma; osteogenesis promotion at a defect part resulting from an auto-bone grafting for treating osteogenesis promotion after abrating a focus of an osteomyelitis, a bone/joint infectious disease, a chronic joint disease, a metabolic bone disease, a traumatic bone disease or an osteosarcoma; osteogenesis promotion of a skull defect part or a fenestra in a bone; osteogenesis promotion in a tympanoplastics.

The amount of the sustained-release paste of the present invention may be optionally selected depending on an animal in interest, a site to be applied and its purpose. In addition, to the selected amount of the paste to be applied is an effective amount of an osteogenesis promoter. For example, when Compound A is applied to transcervical fracture, the amount of Compound A to be applied per site is about 1 mg to 500 mg, preferably about 5 mg to 300 mg for an active component, and preferably about 0.01 to 50 ml as a volume of the paste.

Other active component than the above osteogenesis promoter may also be added to the sustained-release paste of the present invention, as long as it does not affect the properties of the paste. Such as an active component includes, for example, antibiotics, antineoplastic agents, anti-inflammatory agents, analgesics and the like.

Such antibiotics include, for example, aminoglycoside antibiotics such as amikacin, dibekacin, gentamicin or derivatives thereof.

Such antineoplastic agents include, for example, anthracylin carcinostatic agents such as taxol, doxorubicin hydorchloride, mtothotrexate, etoposide, 5-fluorouracil, mitoxantrone, mesna, dimesna, aminoglutethimide, tamoxifen, acrolein, cisplatin, carboplatin, cyclophosphamide, lomustine (CCNU) , carmustine (BCNU) and the like, or derivatives thereof.

Such anti-inflammatory agents includes, for example, a salicylic acid anti-inflammatory agent such as aspirin, a pyrazolone anti-inflammatory agent such as aminopyrine, an aniline anti-inflammatory agent such as acetaminophen, a pyrazolidinedione anti-inflammatory agent such as phenylbutazone, ketophenylbutazone and the like, an anthranilic acid anti-inflammatory agent such as mefenamic acid, an acetic acid anti-inflammatory agent such as indomethacine, a trioxopyrimidine anti-inflammatory agent such as bucolome, a basic anti-inflammatory agent such as benzydamin, mepirizole, tiaramide, tinoridine, an anti-inflammatory enzyme preparation, a nonsteroidal aniti-inflammatory agent.

Such analgesics include, for example, xylocaine.

The main base material of sustained-release paste of the present invention, a calcium component, is non-toxic and highly compatible to a bone tissue. Therefore, the compositions of the invention are lesser toxic and can be used safely mammals (e.g., human, mouse, rat, rabbit, dog, cat, bovine, equine, porcine, etc.). In addition, since a calcium component, which is the base material, is radiopaque, the paste can be used to confirm the site where it has been filled. Thus, the paste is very useful clinically. (See the following Reference Example.)

Since the sustained-release paste of the present invention shows good spreadability, application property and plasticity and the like, it is excellent in operativity and handling properties when it is used. Furthermore, since it has a certain hardness, it has high adherability and a high retention to bones, and, therefore, it is suitable for local administration. In addition, the paste of the present invention can be used as a spacer for redressement in a fracture operation, or as a glue or an adhessive to retain broken bone pieces or an artificial bone granules used as a filing material at a fracture site or a bone defect site.

In addition, the sustained-release paste of the present invention can further incorporate an osteogenesis promoter and other physiologically active component therein after increasing its hardness by well mixing, which, in turn, is applied to a fracture site. It is also possible that an osteogenesis promoter or other physiologically active component is attached on the surface of the sustained-release paste of the present invention which has been mixed well, which is applied to a fracture site. The above variations of application allow separately administering desired physiologically active components to a site to be treated at predetermined moments.

The administration of the sustained-release paste of the present invention can greatly reduce a healing time because an osteogenestic promotion effect of the osteogenesis promoter progresses effectively. As a result, it becomes possible to enhance a return to the social life of the patient, or to prevent a variety of complications accompanying fracturing in senescence from occurring. Thus, the sustained-release paste of the present invention is a very useful medicament in a surgical treatment of a fracture.

### Example

The following Reference Example, Examples and Comparative Examples, as well as their comparative test results, describe the present invention in more detail, but the invention is not limited to these descriptions.

Compound A used below was produced according to the method as described in JP-A 8-231569 (EP-A 719782).

### Reference Example

In order to obtain a sustained-release preparation of Compound A, a microcapsule was prepared according to a method described in JP-A 9-236545 (WO 96/39134). Specifically, 0.55 g of copolymer of lactic acid-glycolic acid (lactic acid : glycolic acid = 85 : 15 (molar ratio); weight-average molecular weight 14,000) and 4.45 g of Compound A were dissolved in 8 mL of dichloromethane to yield an oil phase. This solution was quickly poured into 800 mL of 0.1% poly (vinyl alcohol) solution (15 °C) to yield an o/w emulsion. This was further stirred at room temperature for 3 hours to evaporate dichloromethane. The resulting solidified microcapsules were collected by centrifugation and washed with distilled water. Mannitol (0.6 g) was added, and the mixture was dispersed to suspend in a small amount of water and lyophilized. The resulting microcapsules were further dried at 42°C *in vacuo* to completely remove the residual dichloromethane.

### Example 1

480 mg (containing 48 mg of Compound A) of microcapsule prepared according to a method described in Reference Example was mixed with a powder comprising tetracalcium phosphate (TRADE NAME: TTCP, produced by Taihei Chemical Industrial Co., Ltd.) 544 mg, dicalcium phosphate dihydrate (produced by Wako Pure Chemical Industries, Ltd.) 256mg, and dextran (TRADE NAME: T70, produced by Pharmacia Biotech Inc., average molecular weight 70,000) 400 mg, which was then kneaded with 0.4 ml of phosphate buffer solution (0.2 M, pH 7.4) containing 0.5 % (w/v) sodium carboxymethylcellulose to yield the sustained-release paste of the present invention.

### Example 2

480 mg (containing 48 mg of Compound A) of microcapsule prepared according to a method described in Reference Example was mixed with a powder comprising α-tricalcium phosphate (TRADE NAME: α-TCP, produced by Taihei Chemical Industrial Co., Ltd.) 760 mg,dicalcium phosphate dihydrate (produced by Wako Pure Chemical Industries, Ltd.) 40 mg, and dextran (TRADE NAME: T70, produced by Pharmacia Biotech Inc., average molecular weight 70,000) 400 mg, which was then kneaded with 0.4 ml of phosphate buffer solution (0.2 M, pH7.4) containing 0.5 % (w/v) sodium carboxymethylcellulose to yield the sustained-release paste of the present invention.

### Example 3

480 mg (containing 48 mg of Compound A) of microcapsule prepared according to a method described in Reference Example was mixed with a powder comprising tetracalcium phosphate (TRADE NAME: TTCP, produced by Taihei Chemical Industrial Co., Ltd.) 544 mg, dicalcium phosphate dihydrate (produced by Wako Pure Chemical Industries, Ltd.) 256 mg, dextran (TRADE NAME: T70, produced by Pharmacia Biotech Inc., average molecular weight 70,000) 400 mg, and hydroxypropylcellulose (TRADE NAME: HPC-H, produced by Nippon Soda Co., Ltd.) 100 mg, which was then kneaded with 0.4 ml of phosphate buffer solution (0.2 M, pH7.4) containing 0.5 % (w/v) sodium carboxymethylcellulose to yield the sustained-release paste of the present invention.

### Example 4

480 mg (containing 48 mg of Compound A) of microcapsule prepared according to a method described in Reference Example was mixed with a powder comprising tetracalcium phosphate (TRADE NAME: TTCP, produced by Taihei Chemical Industrial Co., Ltd.) 544 mg, dicalcium phosphate dihydrate (produced by Wako Pure Chemical Industries, Ltd.) 256 mg, dextran (TRADE NAME: T70, produced by Pharmacia Biotech Inc., average molecular weight 70,000) 400 mg, and xylitol (produced by Waco Pure Chemical Industries, Ltd.) 40 mg, which was then kneaded with 0.4 ml of phosphate buffer solution (0.2 M, pH7.4) containing 0.5 % (w/v) sodium carboxymethylcellulose to yield the sustained-release paste of the present invention.

### Example 5

480 mg (containing 48 mg of Compound A) of microcapsule prepared according to a method described in Reference Example was mixed with a powder comprising tetracalcium phosphate (TRADE NAME: TTCP, produced by Taihei Chemical Industrial Co., Ltd.) 544 mg, dicalcium phosphate dihydrate (produced by Wako Pure Chemical Industries, Ltd.) 256 mg, dextran (TRADE NAME: T70, produced by Pharmacia Biotech Inc., average molecular weight 70,000) 400 mg, and xylitol (produced by Waco Pure Chemical Industries, Ltd.) 40 mg, which was then kneaded with 0.4 ml of phosphate buffer solution (1.0 M, pH7.4) to yield the sustained-release paste of the present invention.

### Example 6

480 mg (containing 48 mg of Compound A) of microcapsule prepared according to a method described in Reference Example was mixed with a powder comprising tetracalcium phosphate (TRADE NAME: TTCP, produced by Taihei Chemical Industrial Co., Ltd.) 544 mg, dicalcium phosphate dihydrate (produced by Wako Pure Chemical Industries, Ltd.) 256 mg, dextran (TRADE NAME: T70, produced by Pharmacia Biotech Inc., average molecular weight 70,000) 400 mg, and xylitol (produced by Wako Pure Chemical Industries, Ltd.) 100 mg, which was then kneaded with 0.4 ml of phosphate buffer solution (0.2 M, pH7.4) containing 0.5 % (w/v) sodium carboxymethylcellulose to yield the sustained-release paste of the present invention.

### Example 7

480 mg (containing 48 mg of Compound A) of microcapsule prepared according to a method described in Reference Example was mixed with a powder comprising tetracalcium phosphate (TRADE NAME: TTCP, produced by Taihei Chemical Industrial Co., Ltd.) 752 mg, calcium carbonate (produced by Wako Pure Chemical Industries, Ltd.)208 mg, dicalcium phosphate dihydrate (produced by Wako Pure Chemical Industries, Ltd.) 640 mg, and dextran (TRADE NAME: T70, produced by Pharmacia Biotech Inc., average molecular weight 70, 000) 400 mg, which was then kneaded with 0.4 ml of phosphate buffer solution (0.2 M, pH7.4) containing 0.5 % (w/v) sodium carboxymethylcellulose to yield the sustained-release paste of the present invention.

### Example 8

480 mg (containing 48 mg of Compound A) of microcapsule prepared according to a method described in Reference Example was mixed with a powder comprising tetracalcium phosphate (TRADE NAME: TTCP, produced by Taihei Chemical Industrial Co., Ltd.) 544 mg, dicalcium phosphate dihydrate (produced by Wako Pure Chemical Industries, Ltd.) 256 mg, dextran (TRADE NAME: T70, produced by Pharmacia Biotech Inc., average molecular weight 70, 000) 400 mg, and sodium hydrogencarbonate (produced by Wako Pure Chemical Industries, Ltd.) 100 mg, which was then kneaded with 0.4 ml of phosphate buffer solution (1.0 M, pH7.4) to yield the sustained-release paste of the present invention.

### Example 9

480 mg (containing 48 mg of Compound A) of microcapsule prepared according to a method described in Reference Example was mixed with a powder comprising tetracalcium phosphate (TRADE NAME: TTCP, produced by Taihei Chemical Industrial Co., Ltd.) 544 mg, dicalcium hphosphate dihydrate (produced by Wako Pure Chemical Industries, Ltd.) 256 mg, dextran (TRADE NAME: T70, produced by Pharmacia Biotech Inc., average molecular weight 70,000) 400 mg, and hydroxypropylcellulose (TRADE NAME: HPC-H, produced by Nippon Soda Co., Ltd.) 100 mg, which was then kneaded with 0.4 ml of phosphate buffer solution (1.0 M, pH7.4) to yield the sustained-release paste of the present invention.

### Example 10

480 mg (containing 48 mg of Compound A) of microcapsule prepared according to a method described in Reference Example was mixed with a powder comprising calcium sulfate hemihydrate (produced by Wako Pure Chemical Industries, Ltd.) 800 mg, and dextran (TRADE NAME: T70, produced by Pharmacia Biotech Inc., average molecular weight 70, 000) 400 mg, which was then kneaded with 0.4 ml of saline to yield the sustained-release paste of the present invention.

### Example 11

480 mg (containing 48 mg of Compound A) of microcapsule prepared according to a method described in Reference Example was mixed with a powder comprising calcium sulfate hemihydrate (produced by Wako Pure Chemical Industries, Ltd.) 800 mg, dextran (TRADE NAME: T70, produced by Pharmacia Biotech Inc., average molecular weight 70,000) 400 mg, and hydroxypropylcellulose (TRADE NAME: HPC-H, produced by Nippon Soda Co., Ltd.) 100 mg, which was then kneaded with 0.4 ml of physiological saline to yield the sustained-release paste of the present invention.

### Example 12

480 mg (containing 48 mg of Compound A) of microcapsule prepared according to a method described in Reference Example was mixed with a powder comprising calcium sulfate hemihydrate (produced by Wako Pure Chemical Industries, Ltd.) 400 mg, dextran (TRADE NAME: T70, produced by Pharmacia Biotech Inc., average molecular weight 70,000) 400 mg, and hydroxyapatite (TRADE NAME: HAP-200, produced by Taihei Chemical Industrial Co., Ltd., the specific surface area 1-2 m²/g) 400 mg, which was then kneaded with 0.4 ml of saline to yield the sustained-release paste of the present invention.

### Example 13

480 mg (containing 48 mg of Compound A) of microcapsule prepared according to a method described in Reference Example was mixed with a powder comprising calcium sulfate hemihydrate (produced by Wako Pure Chemical Industries, Ltd.) 400 mg, dextran (TRADE NAME: T70, produced by Pharmacia Biotech Inc., average molecular weight 70,000) 400 mg, and hydroxyapatite (TRADE NAME: HAP-300, produced by Taihei Chemical Industrial Co., Ltd., the specific surface area 50-55 m²/g) 400 mg, which was then kneaded with 0.4 ml of saline to yield the sustained-release paste of the present invention.

### Example 14

480 mg (containing 48 mg of Compound A) of microcapsule prepared according to a method described in Reference Example was mixed with a powder comprising calcium sulfate hemihydrate (produced by Wako Pure Chemical Industries, Ltd.) 800 mg, dextran (TRADE NAME: T70, produced by Pharmacia Biotech Inc., average molecular weight 70,000) 400 mg, and sodium hydrogencarbonate (produced by Wako Pure Chemical Industries, Ltd.) 50 mg, which was then kneaded with 0.4 ml of saline to yield the sustained-release paste of the present invention.

### Example 15

44 mg of crystalline powder of Compound A was mixed with a powder comprising tetracalcium phosphate (TRADE NAME: TTCP, produced by Taihei Chemical Industrial Co., Ltd.) 544 mg, dicalcium phosphate dihydrate (produced by Wako Pure Chemical Industries, Ltd.) 256mg, and dextran (TRADENAME: T70, produced by Pharmacia Biotech Inc., average molecular weight 70,000) 400 mg, which was then kneaded with 0.3 ml of phosphate buffer solution (0.2 M, pH7.4) containing 0.5 % (w/v) sodium carboxymethylcellulose to yield the sustained-release paste of the present invention.

### Comparative Example 1

480 mg (containing 48 mg of Compound A) of microcapsule prepared according to a method described in Reference Example was dispersed in 1.6 ml of dispersion medium (a distilled water in which D-sorbitol 2.5 %(w/v), sodium chloride 0.9 % (w/v), polysorbate 80 0.1 % (w/v), disodium hydrogenphosphate 0.0715 % (w/v), and sodium carboxymethylcellurose 0.5 % (w/v)) to yield a suspension.

### Comparative Example 2

480 mg (containing 48 mg of Compound A) of microcapsule prepared according to a method described in Reference Example was mixed with a powder comprising tetracalcium phosphate (TRADE NAME: TTCP, produced by Taihei Chemical Industrial Co., Ltd.) 544 mg, and dicalcium phosphate dihydrate (produced by Wako Pure Chemical Industries, Ltd.) 256 mg, which was then kneaded with 0.6 ml of phosphate buffer solution (0.2 M, pH7.4) containing 0.5 % (w/v) of sodium carboxymethylcellulose to yield a homogeneous mixture.

### Comparative Example 3

A powder comprising tetracalcium phosphate (TRADE NAME: TTCP, produced by Taihei Chemical Industrial Co., Ltd.) 544 mg and dicalcium phosphate dihydrate (produced by Wako Pure Chemical Industries, Ltd.) 256 mg was kneaded with 0.4 ml of phosphate buffer solution (0.2 M,pH7.4) containing 1.0 % (w/v) of sodium alginate (produced by Wako Pure Chemical Industries, Ltd.) to yield a homogeneous mixture.

### Comparative Example 4

A powder comprising tetracalcium phosphate (TRADE NAME: TTCP, produced by Taihei Chemical Industrial Co., Ltd.) 544 mg and dicalcium phosphate dihydrate (produced by Wako Pure Chemical Industries, Ltd.) 256 mg was kneaded with 0.4 ml of phosphate buffer solution (0.2 M, pH7.4) containing 2.0 % (w/v) of atelocollagen (produced by Funakoshi Co., Ltd.) to yield a homogeneous mixture.

### Comparative Example 5

44 mg of crystalline powder of Compound A was mixed with a powder comprising tetracalcium phosphate (TRADE NAME: TTCP, produced by Taihei Chemical Industrial Co., Ltd.) 544 mg and dicalcium phosphate dihydrate (produced by Wako Pure Chemical Industries, Ltd.) 256 mg, which was then kneaded with 0.5 ml of phosphate buffer solution (0.2 M, pH7.4) containing 0.5 % (w/v) of sodium carboxymethylcellulose to yield a homogeneous mixture.

### Comparative Test 1: Evaluation of Retention

The sustained-release paste obtained in Example 1 (0.16 ml; containing about 5 mg of Compound A) was administered to a lateral femur of a male SD rat (10 weeks old) 20 minutes after mixing. The administered site was, then, opened 2 hours, 1 day and 1 week after administration to examine the existence of the remaining sustained-release paste for evaluating retention after local administration.

The observation demonstrated that all of these sustained-release paste did not disintegrate even when they contacted with a body fluid such as blood and they sufficiently remained at the administration site even 1 week after administration.

In contrast, the suspension obtained in Comparative Example 1 (0.16 ml, containing about 5 mg of Compound A) was administered to a lateral femur similarly. Evaluation of the retention after local administration revealed that the suspension flew away immediately after administration, showing no retention.

### Comparative Test 2: Verification of Osteogenesis

The sustained-release paste obtained in Example 1 (0.3 ml, containing about 9.4 mg of Compound A) was administered to a hole with a diameter of 5 mm, which was made in the center of a lateral tibia in one leg of a Japanese white rabbit and the periphery thereof 15 minutes after mixing. As a control, a mixture made by kneading a calcium phosphate powder having 0.3 ml of composition identical to that obtained in Example 1 with a PLGA microcapsule (480 mg, containing no Compound A) was administered to a hole in a lateral tibia in the other leg. After administration, the progresses of osteogenesis were compared by X-ray radiography at around the holes in the tibiae in the course of time.

Both in the control and in the side administered with Compound A, a high retention of the paste at the administered site from immediately after administration through the end of the test by X-ray radiography. Figures 1 and 2 show radiographs around the holes in the tibiae after 7 weeks. In the control (Fig. 1), osteogenesis was not observed, whereas a significant osteogenesis by membranous ossification was observed in the side administered with Compound A (Fig. 2).

### Comparative Test 3: Verification of Sustained Releasability

The sustained-release paste obtained in Example 1 (0.16 ml, containing about 5 mg of Compound A) was subcutaneously implanted into the backs of male SD rats (6 week old) 10 minutes after mixing (n=4). Rats were sacrificed in every certain interval after implantation to remove the remaining paste at the implanted site, and the amounts of Compound A and PLGA which is a component of the microcapsule incorporating Compound Awere measured by HPLC.

Change in the residual ratio of Compound A is shown in Figure 3. As shown in Fig. 3, it is indicated that Compound A was released gradually over 3 months. On the other hand, PLGA almost disappeared after 2 month.

### Comparative Test 4: Change in Blood level

The sustained-release paste obtained in Example 1 (0.16 ml, containing about 5 mg of Compound A) was subcutaneously implanted into the backs of male SD rats (6 week old) 10 minutes after mixing (n=4) . Blood was taken from tail veins of the rats in every certain interval after implantation and the concentration of Compound A in the serum was determined by an enzyme immunoassay (EIA) .

Change in the concentration of Compound A in the serum is shown in Figure 4. As shown in Fig. 4, Compound A was detected in the serum over 3 months. On the other hand, a similar test for a microcapsule alone of Compound A, its period for sustained releasing was 1 month. It was verified that mixing a calcium component with a hydraulic setting property with the microcapsule prolonged the period for sustained releasing relative to that for the microcapsule alone.

### Comperative Test 5: Estimation of Properties

A setting time, a compressive strength, a diametral tensile strength, adherability, and a distribution of pores for each of pastes obtained in the above Examples and Comparative Examples were estimated.

The setting time as used herein is measured by a Vicat indentation test according to a measurement method for a dental Zinc phosphate cement (JIS T6602). Specifically, the mixture is transported to a thermostatic chamber at 37 °C at about 100 % of a relative humidity 3 minutes after the start of mixing. Then, a 300 g of a Vicat needle is gently dropped onto the surface of the mixture to check whether a needle mark is made. A time from the start of mixing to a time where a needle mark is not made any more is defined as a setting time.

The compressive strength is obtained by measuring a strength of set compositions (formed into a cylinder with a diameter of 1.0 cm and a height of 1.0 cm) using a universal testing machine (INTESCO Model 205E) 14 hours and two weeks after mixing under a condition at 37 °C and a relative humidity of about 100 %.

The diametral tensile strength (DTS) is measured using a set composition formed into a cylinder with a diameter of 1.0 cm and a height of 0.5 cm under the same condition for the compressive strength.

The adhesion force is obtained by putting a 0.1 ml of the mixture within two glass plates 5 minutes after mixing and by measuring a strength to peel the two glass plates 1 minute after holding the pressure at 29.4 N using the above universal testing machine (INTESCO Model 205E).

The distribution of pores was measured by mercury porosimetry (AutoPore II 9220, SHIMADZU Co., Ltd.).

Results are summarized in the following table.

| Composition | Setting Time | Compressive Strength after 14 h (MPa) | Compressive Strength after 2 weeks (MPa) | DTS after 14 h (MPa) | Adhesion Force (N) |
|---|---|---|---|---|---|
| Example 1 | 3 h | 0.8 | 1.7 | 0.1 | 12.2 |
| Example 3 | 4 h | 0.5 | 1.3 | 0.09 | 12.8 |
| Example 4 | 6 h | NT | NT | NT | NT |
| Example 5 | 11 h | NT | NT | NT | NT |
| Example 9 | 11 h | 0.4 | 1.2 | 0.06 | 11.2 |
| Example 15 | 9 h | 0.7 | NT | 0.1 | 9.3 |
| Comp.Ex. 2 | 32 min. | 1.7 | 3.5 | 0.5 | 0.9 |
| Comp.Ex. 3 | 13 min. | 3.3 | 3.5 | 0.9 | 0.6 |
| Comp.Ex. 4 | 20 min. | 4.1 | NT | 1.2 | 5.7 |

As shown in the table, the setting times for all of the sustained-release paste of the present invention are over 1 hour, indicating significant differences from those for mixtures incorporating sodium alginate or collagen in Comparative Example 3 or 4. In addition, the compressive strengths after 14 hours for all of the sustained-release paste of the present invention are less than 1.5 MPa, indicating that they have lower strengths than the mixtures incorporating sodium alginate or collagen in Comparative Example 3 or 4.

As it is clearly indicated above, it is verified that all of compositions of Comparative Examples which have smaller compositional ratio of a viscosity-increasing agent relative to the range of the present paste shows a shorter setting time and higher compressive strength after setting. This is because the compositions in Comparative Examples are designed aiming to an artificial bone and a bone repairing material which is required to have a high mechanical strength. On the other hand, the sustained-release paste of the present invention, which is required to have spreadability and an application property, retains required palasticity for a long period. In addition, the adhesion force of the sustained-release paste of the present invention is stronger than that of any one of the compositions in Comparative Examples, indicating excellent adherability of the present sustained-release paste.

Further, in the distributions of pores in the set sustained-release pastes of the present invention, many pores existed at a pore size in the range around 1 µm to 20 µm.

### Example 16

480 mg (containing 48 mg of Compound A) of microcapsule prepared according to a method described in Reference Example was mixed with a powder comprising α-calcium sulfate hemihydrate (produced by SAN ESU Gypsum Co., Ltd.) 400 mg, and dextran (TRADE NAME: T70, produced by Pharmacia Biotech Inc., average molecular weight 70, 000) 400 mg, which was then kneaded with 0.2 ml of phosphate buffered saline (produced by Takara Shuzo Co., Ltd.) to yield the sustained-release paste of the present invention.

### Example 17

480 mg (containing 48 mg of Compound A) of microcapsule prepared according to a method described in Reference Example was mixed with a powder comprising β-calcium sulfate hemihydrate (produced by SAN ESU Gypsum Co., Ltd.) 400 mg, and dextran (TRADE NAME: T70, produced by Pharmacia Biotech Inc., average molecular weight 70,000) 400 mg, which was then kneaded with 0.24 ml of phosphate buffered saline (produced by Takara Shuzo Co., Ltd.) to yield the sustained-release paste of the present invention.

### Example 18

480 mg (containing 48 mg of Compound A) of microcapsule prepared according to a method described in Reference Example was mixed with a powder comprising α-calcium sulfate hemihydrate (produced by SAN ESU Gypsum Co., Ltd.) 200 mg, calcium sulfate dihydrate (produced by Wako Pure Chemical Industries, Ltd.), and dextran (TRADE NAME: T70, produced by Pharmacia Biotech Inc., average molecular weight 70,000) 400 mg, which was then kneaded with 0.2 ml of phosphate buffered saline (produced by Takara Shuzo Co., Ltd.) to yield the sustained-release paste of the present invention.

### Example 19

480 mg (containing 48 mg of Compound A) of microcapsule prepared according to a method described in Reference Example was mixed with a powder comprising β-calcium sulfate hemihydrate (produced by SAN ESU Gypsum Co., Ltd.) 200 mg, calcium sulfate dihydrate (produced by Wako Pure Chemical Industries, Ltd.), and dextran (TRADE NAME: T70, produced by Pharmacia Biotech Inc., average molecular weight 70,000) 400 mg, which was then kneaded with 0.24 ml of phosphate buffered saline (produced by Takara Shuzo Co., Ltd.) to yield the sustained-release paste of the present invention.

### Example 20

44 mg of crystalline powder of Compound A was mixed with a powder comprising tetracalcium phosphate (TRADE NAME: TTCP, produced by Taihei Chemical Industrial Co., Ltd.) 544 mg, dicalcium phosphate dihydrate (produced by Wako Pure Chemical Industries, Ltd.) 256mg, and dextran (TRADENAME: T70, produced by Pharmacia Biotech Inc., average molecular weight 70,000) 400 mg, which was then kneaded with 0.74 ml of phosphate buffer solution (0.2 M, pH7.4) containing 0.5 % (w/v) of sodium carboxymethylcellulose to yield the sustained-release paste of the present invention.

### Example 21

44 mg of crystalline powder of Compound A was mixed with a powder comprising tetracalcium phosphate (TRADE NAME: TTCP, produced by Taihei Chemical Industrial Co., Ltd.) 544 mg, dicalcium phosphate dihydrate (produced by Wako Pure Chemical Industries, Ltd.) 256 mg, and dextran (TRADE NAME: T70, produced by Pharmacia Biotech Inc., average molecular weight 70,000) 1000 mg, which was then kneaded with 1.09 ml of phosphate buffer solution (0.2 M, pH7.4) containing 0.5 % (w/v) of sodium carboxymethylcellulose to yield the sustained-release paste of the present invention.

### Test 1: Verification of Sustained Releasability

The sustained-release pastes obtained in Examples 16-19 (0.14 ml of each, containing about 5 mg of Compound A) were subcutaneously implanted into the backs of male SD rats (6 week old) 20 minutes after mixing (n=4). Rats were sacrificed in every certain interval after implantation to remove the remaining paste at the implanted site, and the amounts of Compound A was measured by HPLC.

Change in the residual ratio of Compound A is shown in Figure 5. As shown in Fig. 5, it is indicated that Compound A was released gradually over 1 month.

### Test 2: Change in Blood level

The sustained-release paste obtained in Example 18 (0.14 ml, containing about 5 mg of Compound A) was subcutaneously implanted into the backs of male SD rats (6 week old) 20 minutes after mixing (n=4). Blood was taken from tail veins of the rats in every certain interval after implantation and the concentration of Compound A in the serum was determined by an enzyme immunoassay (EIA).

Change in the concentration of Compound A in the serum is shown in Figure 6. As shown in Fig. 6, Compound A was detected in the serum over 1 month.

### Test 3: Verification of Sustained Releasability

The sustained-release paste obtained in Examples 18 (0.6 ml, containing about 21.4 mg of Compound A) was subcutaneously implanted into the backs of male SD rats (6 week old) 20 minutes after mixing (n=4). Rats were sacrificed in every certain interval after implantation to remove the remaining paste at the implanted site, and the amounts of Compound A was measured by HPLC.

Change in the residual ratio of Compound A is shown in Figure 7. As shown in Fig. 7, it is indicated that Compound A was released gradually over 1 month.

### Test 4: Change in Blood level

The sustained-release paste obtained in Example 18 (0.6 ml, containing about 21.4 mg of Compound A) was subcutaneously implanted into the backs of male SD rats (6 week old) 20 minutes after mixing (n=4) . Blood was taken from tail veins of the rats in every certain interval after implantation and the concentration of Compound A in the serum was determined by an enzyme immunoassay (EIA).

Change in the concentration of Compound A in the serum is shown in Figure 8. As shown in Fig.8, Compound A was detected in the serum over 1 month.

### Test 5: Verification of Osteogenesis

The sustained-release paste obtained in Example 18 (0.6 ml, containing about 21.4 mg of Compound A) was administered to a hole with a diameter of 5 mm, which was made in the center of a lateral tibia in one leg of a Japanese white rabbit and the periphery thereof 20 minutes after mixing. As a control, 0.6 ml of mixture made by kneading a calcium sulfate powder having a composition identical to that obtained in Example 18 with 480 mg of microcapsule of a copolymer of lactic acid-glycolic acid (lactic acid : glycolic acid = 85 : 15 (molar ratio); weight-average molecular weight 14,000) (containing no Compound A) was administered to a hole in a lateral tibia in the other leg. After administration, the progresses of osteogenesis were compared by X-ray radiography at around the holes in the tibiae in the course of time.

As a result of the observation of radiographs around the holes in the tibiae after 2 and 4 weeks, both in the control and in the side administered with Compound A, a high retention of the past at the administered site from immediately after administration through 2 weeks after administration. Four weeks after administration, osteogenesis was not observed in the control, whereas a significant osteogenesis by membranous ossification was observed in the side administered with Compound A.

### Test 6

The mixture obtained in Comparative Example 5 (0.09 ml, containing about 5 mg of Compound A), the paste obtained in Example 20 (0.13 ml, containing about 5 mg of Compound A), and the paste obtained in Example 21 (0.20 ml, containing about 5 mg of Compound A) were individually added to 900 ml of test solutions comprising 20 mM phosphate/15 mM acetate buffer solution (pH 7.0) containing 0.3 % hexadecyltrimethylammonium bromide to carry out an *in vitro* release test via a paddle method. The rotation speed was 100 rpm and the temperature of the test solution was 47 °C. The test solution was removed in the course of time to measure the amount of Compound A released from the pastes or the mixture. A release ratio (%) is represented by a ratio of the amount of released Compound A to the initial amount of Compound A. The result is shown in Figure 9.

The amount of released Compound A depended on the amount of dextran to be added, larger amount of dextran to be added showed higher release ratio of Compound A.

### Industrial Applicability

Since the sustained-release paste of the present invention has an excellent osteoconductivity, adheres to a bone well, and can release gradually an osteogenesis promoter, it can be applied as a bone repairing material for a fracture or a bone defect site, and as a bone coaptation material in a bone transplantation or in a replacement arthroplasty, alternatively, in the regimen of an oral surgery, it can be applied for palingenesis of a lost alveolus or increase in the amount of an alveolus. Therefore, it is clinically very useful. In addition, since the sustained-release paste of the present invention can be used together with an artificial bone, an artificial joint, a bone filling material, a bone substitution material, a bone coaptation material and the like, it can greatly reduce a healing time for a bone disease such as a fracture, which requires an operation.

As a result, it becomes possible to enhance a return to the social life of the patient, or to prevent a variety of complications accompanying fracturing in senescence from occurring.

## Claims

1. An osteogenesis promoter sustained-release paste, which comprises an osteogenesis promoter, a calcium component and a viscosity-increasing agent.

2. The paste according to claim 1, wherein the calcium component is selected from a group consisting of calcium phosphate, sulfate and carbonate, and any combination thereof.

3. The paste according to claim 1, wherein the calcium component is a combination of an anhydrate or a dihydrate of dicalcium phosphate and tetracalcium phosphate.

4. The paste according to claim 1, wherein the calcium component is a hemihydrate of calcium sulfate.

5. The paste according to claim 4, which further comprises a dihydrate of calcium sulfate.

6. The paste according to claim 1, wherein the calcium component is incorporated in about 1-500 parts relative to a part of the osteogenesis promoter by weight.

7. The paste according to claim 1, wherein the viscosity-increasing agent is a biocompatible polymer.

8. The paste according to claim 1, wherein the viscosity-increasing agent is dextran.

9. The paste according to claim 1, wherein the viscosity-increasing agent is incorporated in about 0.05-4 parts relative to a part of the calcium component by weight.

10. The paste according to claim 1, wherein the osteogenesis promoter is a non-peptide low-molecular-weight compound.

11. The paste according to claim 1; wherein the osteogenesis promoter is benzothiopyran or a benzothiepine derivative.

12. The paste according to claim 1, wherein the osteogenesis promoter is (2*R*,4*S*)- (-) -*N*- [4-(diethoxyphosphorylmethyl)phenyl]-1,2,4,5-tetrahydro-4-methyl-7,8-methylenedioxy-5-oxo-3-benzothiepine-2-carboxamide or a salt thereof, or a sustained-release preparation thereof.

13. The paste according to claim 1, wherein the osteogenesis promoter is formulated so as to have sustained-releasability with a biodegradative polymer.

14. A composition for producing the paste according to claim 1, which comprises a calcium component and a viscosity-increasing agent.

15. A composition for producing the paste according to claim 1, which comprises an osteogenesis promoter or a sustained-release preparation thereof, a calcium component and a viscosity-increasing agent.

16. A process for producing an osteogenesis promoter sustained-release paste, which comprises mixing an osteogenesis promoter, a calcium component, a viscosity-increasing agent and water.

17. The paste according to any one of claims 1 to 13, which is a bone disease treatment agent.

18. A process for treating a bone disease, which comprises administering the paste according to any one of claims 1 to 13.

19. Use of the composition according to claim 14 or 15 for the manufacture of a bone disease treating agent.
